# EUROPEAN PATENT SPECIFICATION

(11) **EP 0 625 508 B1**
(45) Date of publication and mention of the grant of the patent: **19.08.1998**
(21) Application number: 94107448.6
(22) Date of filing: 13.05.1994
(51) Int. Cl.: C07C 323/32, C07C 317/26, A01N 31/08, A01N 37/34, A01N 41/10

(54) **Sulfur-containing derivatives of 2-cyano-1,3-diones and their use as herbicides**
Schwefelenthaltende 2-Cyan-1,3-Dion-Derivate und ihre Verwendung als Herbizid
Dérivés de 2-cyano-1,3 dione contenant du soufre et leur emploi en tant qu'herbicides

(30) Priority: 18.05.1993 GB 9310203
(43) Date of publication of application: 23.11.1994
(73) Proprietor: RHONE POULENC AGRICULTURE LTD., Ongar, Essex CM5 OHW (GB)
(72) Inventor: Cramp, Susan Mary, Rhone Poulenc, Ongar, Essex CM5 0HW (GB); Wallis, Derek Ian, Rhone Poulenc, Ongar, Essex CM5 0HW (GB); Lambert, Claude, Ibaraki 304 (JP); Yarwood, Thomas David, Rhone Poulenc, Ongar, Essex CM5 0HW (GB)
(74) Representative: Brachotte, Charles

(56) References cited:
- EP-A- 0 213 892
- EP-A- 0 496 630
- EP-A- 0 496 631

## Description

This invention relates to novel 2-cyano-1,3-dione derivatives, processes for their preparation, compositions containing them, and their use as herbicides.

EP-A-0213892 discloses herbicidally active enol compounds and specifically discloses certain 2-cyano-1,3-dione derivatives. EP-A-0496630 and 0496631 describe certain 2-cyano-1,3-diones as herbicides which contain a sulphur substituent at the 4-position and 2-position respectively of the benzoyl moiety.

The present invention provides 2-cyano-1,3-diones of formula I: 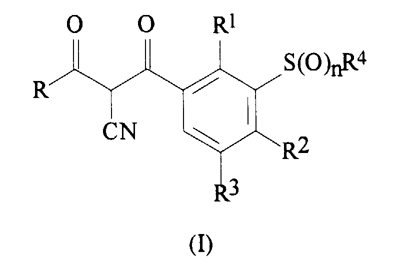 wherein:
R represents cyclopropyl;
R¹ represents:-
   a hydrogen, chlorine or bromine atom, or
   a straight- or branched-chain alkyl group containing up to 6 carbon atoms which is substituted by -OR⁵; or
   a group selected from R⁵, nitro, cyano, -S(O)ₚR⁵, -OR⁵, -O(CH₂)ₘOR⁵ and -CO₂R⁵;
R² and R³, which may be the same or different, each represents: -
   a halogen or hydrogen atom, or
   a straight- or branched-chain alkyl group containing up to 6 carbon atoms which is substituted by -OR⁵; or
   a group selected from R⁵, nitro, cyano, -OR⁵, -O(CH₂)ₘOR⁵, -S(O)_{q}R⁵ and -CO₂R⁵,
R⁴ and R⁵, which may be the same or different, each represents:-
   a straight- or branched-chain all group containing up to 6 carbon atoms which is optionally substituted by one or more halogen atoms which may be the same or different;
   m is an integer from 1 to 3;
   n is zero, 1 or 2; p is zero, 1 or 2; q is zero, 1 or 2;
   with the proviso that when R¹ represents -S(O)ₚR⁵ at least one of the groups p and q is zero;
metal complexes thereof, and agriculturally acceptable salts thereof, which possess valuable herbicidal properties.

Compounds of formula I may exist in enolic tautomeric forms that may give rise to geometric isomers around the enolic double bond.

Furthermore, in certain cases the substituents R, R¹, R², R³, R⁴, and R⁵ may contribute to optical isomerism and/or stereoisomerism. All such forms are embraced by the present invention.

By the term "agriculturally acceptable salts" is meant salts the cations of which are known and accepted in the art for the formation of salts for agricultural or horticultural use. Preferably the salts are water soluble.

Suitable salts formed by compounds of formula I which are acidic, i.e. in enolic tautomeric forms, with bases include alkali metal (e.g. sodium and potassium salts), alkaline earth metal (e.g. calcium and magnesium) salts, ammonium (e.g. dioctylmethylamine and morpholine) salts.

By the term "metal complexes" is meant compounds in which one or both of the oxygen atoms of the 1,3-dione act as chelating agents to a metal cation. Examples of such cations include zinc, manganese, cupric, cuprous, ferric, ferrous, titanium and aluminium.

The compounds of this invention represent in some aspects of their activity, for example their control of important weed species such as Setaria viridis, Setaria faberii, Echinochloa crus-galli, Avena fatua and Alopecurus myosuroides, an improvement over known compounds.

A preferred class of compounds of formula I are those wherein the substituents have the following preferred meanings :
(a) R¹ represents:-
   a hydrogen, chlorine or bromine atom, or
   a group selected from
   -OR⁵, for example methoxy, ethoxy or trifluoromethoxy,
   R⁵, for example methyl or trifluoromethyl,
   nitro, or -SR⁵; and/or
(b) R² and R³, which may be the same or different, each represents:-
   a halogen atom or hydrogen atom,
   a straight- or branched-chain alkyl group containing up to 6 carbon atoms which is substituted by -OR⁵, for example methoxymethyl; or
   a group selected from
   R⁵, for example methyl or trifluoromethyl,
   -OR⁵, for example methoxy, ethoxy or isopropoxy,
   -O(CH₂)ₘOR⁵ where m is 2 or 3, for example 2-ethoxyethoxy or 2-methoxyethoxy.
   -CO₂R⁵, for example carbomethoxy, carboethoxy, or carboisopropoxy; or
   -S(O)_{q}R⁵,
   provided that at least one of the groups R² and R³ represent hydrogen; and/or
(c) R⁴ represents:-
   a straight- or branched-chain alkyl group containing up to 4 carbon atoms which is optionally substituted by one or more halogen atoms, which may be same or different, for example isopropyl, methyl or ethyl; and/or
(d) R⁵ represents:-
   a straight- or branched-chain alkyl group containing up to 4 carbon atoms which is optionally substituted by one or more halogen atoms, which may be the same or different, for example methyl, ethyl, isopropyl or trifluoromethyl; and
(e) 'halogen' represents chlorine, bromine or fluorine.

A further preferred class of compounds of formula I are those in which R³ represents a hydrogen atom.

A further preferred class of compounds of formula I are those wherein:
R¹ represents chlorine, bromine, trifluoromethyl, -SR⁵, methoxy or methyl;
R² represents fluorine, chlorine, bromine, trifluoromethyl, -S(O)_{q}R⁵ or methyl,
R³ represents hydrogen;
R⁴ represents methyl, ethyl or isopropyl;
R⁵ represents methyl, ethyl or n-propyl; and
n is zero, one or two.

A further preferred class of compounds of formula I are those wherein:
R¹ represents hydrogen or most preferably methyl, trifluoromethyl, -SMe or methoxy;
R² represents methyl or methoxy or most preferably chlorine or -SMe.
R³ represents hydrogen;
R⁴ represents methyl, ethyl or propyl; and
n is zero, one or two.

Particularly important compounds of formula I include the following:
1. 2-cyano-3-cyclopropyl-1-(4-methyl-3-methylsulphenylphenyl)propan-1,3-dione;
2. 2-cyano-3-cyclopropyl-1-(4-methyl-3-methylsulphonylphenyl)propan-1,3-dione;
3. 2-cyano-3-cyclopropyl-1-(4-methyl-3-methylsulphinylphenyl)propan-1,3-dione:
4. 2-cyano-3-cyclopropyl-1-(4-chloro-3-ethylsulphenyl-2-methylsulphenylphenyl)propan-1,3-dione;
5. 2-cyano-3-cyclopropyl-1-(4-methoxy-3-methylsulphonylphenyl)propan- 1,3-dione;
6. 2-cyano-3-cyclopropyl-1-(4-chloro-3-methylsulphonylphenyl)propan-1,3-dione;
7. 2-cyano-3-cyclopropyl-1-(4-chloro-2-methyl-3-methylsulphenylphenyl)propan-1,3-dione;
8. 2-cyano-3-cyclopropyl-1-(3,4-bismethylsulphenyl-2-trifluoromethylphenyl)propan-1,3-dione;
9. 2-cyano-3-cyclopropyl-1-(4-chloro-2-tnethoxy-3-methylsulphonylphenyl)propan-1,3-dione; and
10. 2-cyano-3-cyclopropyl-1-(4-chloro-2-methylsulphenyl-3-propylsulphenylphenyl)propan-1,3-dione.

The numbers 1 to 10 are used for reference and identification of these compounds hereinafter.

The compounds of formula I can be prepared as hereinafter described.

In the following description where symbols appearing in formulae are not specifically defined it is to be understood that they are 'as hereinbefore defined' in accordance with the first definition of each symbol in this specification.

It is to be understood that in the description of the following processes the sequences may be performed in different orders and that suitable protecting groups may be required to achieve the compounds sought.

According to a feature of the present invention compounds of formula (I) may be prepared from a compound of formula (II): 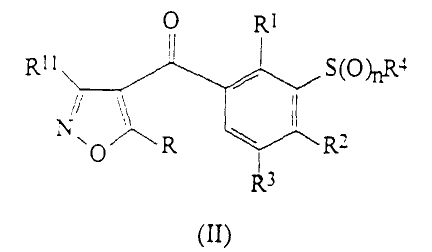 wherein R, R¹, R², R³, R⁴ and n are as hereinbefore defined and R¹¹ represents the hydrogen atom or a group selected from a carboxylic ester, amide, nitrile and acyl.

Where R¹¹ represents hydrogen or an acyl group the reaction is carried out by treatment with a base. Examples of suitable bases include alkali or alkaline earth metal hydroxides or alkoxides such as sodium ethoxide or organic bases such as triethylamine.

Where R¹¹ represents a group such as an ester, amide or nitrile the conversion is carried out by a hydrolytic reaction. The hydrolytic reaction may be carried out in the presence of an acid or base. Acidic hydrolysis may be achieved for example using aqueous hydrochloric acid. Basic hydrolysis may be achieved for example using sodium hydroxide in a mixture of alcohol and water. The reactions are carried out at a temperature between room temperature and the reflux temperature of the mixture.

According to a further feature of the present invention compounds of formula (I) may be prepared from a compound of formula (III): 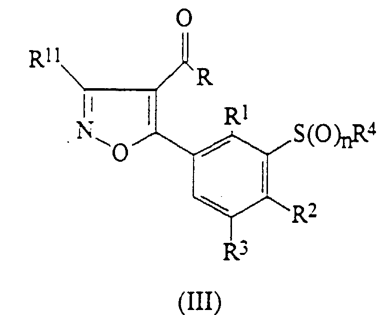 wherein R, R¹, R², R³, R⁴, n and R¹¹ are as hereinbefore defined. Where R¹¹ represents hydrogen or an acyl group the reaction is carried out by treatment with a base. Examples of suitable bases include alkali or alkaline earth metal hydroxides or alkoxides such as sodium ethoxide or organic bases such as triethylamine.

Where R¹¹ represents a group such as an ester, amide or nitrile the conversion is carried out by a hydrolytic reaction. The hydrolytic reaction may be carried out in the presence of an acid or base. Acidic hydrolysis may be achieved for example using aqueous hydrochloric acid. Basic hydrolysis may be achieved for example using sodium hydroxide in a mixture of alcohol and water. The reactions are carried out at a temperature between room temperature and the reflux temperature of the mixture.

According to a further feature of the present invention, compounds of formula (I) in which n, p and q are zero or two may also be prepared by the reaction of a benzoyl chloride of formula (IV): 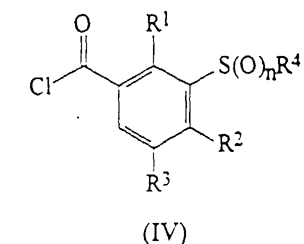 wherein R¹ R², R³ and R⁴ are as hereinbefore defined and n, p and q are zero or two, with a beta-ketonitrile of formula(V): 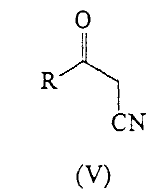 wherein R is as hereinbefore defined. The reaction is generally performed in the presence of a base in a solvent or solvent mixture. Suitable bases include metal hydrides, hydroxides or alkoxides (e.g. sodium or lithim hydride, sodium hydroxide, potassium hydroxide, magnesium ethoxide or magnesium methoxide). Suitable solvents include for example tetrahydrofuran; hydrocarbons such as toluene; or halogenated hydrocarbons such as dichloromethane. The reaction is generally performed at a temperature from 0°C to reflux temperature.

According to a further feature of the present invention, compounds of formula (I) in which n, p and q are zero or two may also be prepared by the reaction of an acid chloride of formula (VI): 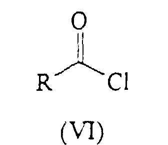 wherein R is as hereinbefore defined, with a beta-ketonitrile of formula (VII): 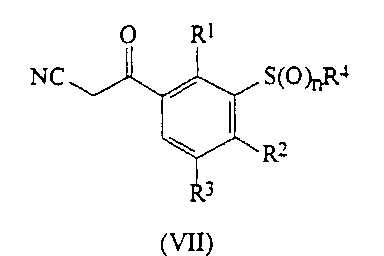 wherein R¹, R², R³, R⁴ are as hereinbefore defined and n, p and q are zero or two. The reaction is generally performed under the same conditions as described above for the reaction of compounds of formula (IV) with compounds of formula (V).

According to a further feature of the present invention compounds of formula (I) in which n, p and q are zero or two may be prepared by the reaction of a benzoyl chloride of formula (IV) wherein R¹, R², R³ and R⁴ are as hereinbefore defined and n, p and q are zero or two, with a beta-ketonitrile of formula (V) wherein R is as hereinbefore defined, via an intermediate of formula (VIII): 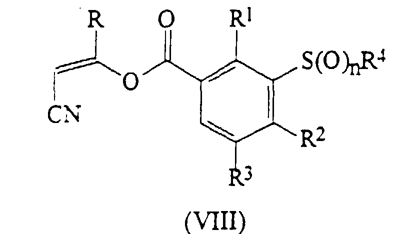 wherein R, R¹, R², R³, R⁴ are as hereinbefore defined and n, p and q are zero or two. The formation of the intermediate of formula (VIII) may be carried out in the presence of a mild base such as an organic base e.g. triethylamine, in an inert solvent such as acetonitrile or dichloromethane at a temperature between room temperature and the reflux temperature of the mixture. The rearrangement of the intermediate of formula (VIII) to a compound of formula (I) may be carried out optionally in situ in an inert solvent such as acetonitrile or dichloromethane in the presence of a catalyst such as a source of cyanide. Examples of such sources of cyanide are acetone cyanohydrin or an alkali metal cyanide such as potassium cyanide, optionally in the presence of a crown ether such as 18-crown-6.

According to a further feature of the present invention compounds of formula (I) in which n, p and q are zero or two may be prepared by the reaction of an acid chloride of formula (VI) wherein R is as hereinbefore defined, with a beta-ketonitrile of formula (VII) wherein R¹, R², R³ and R⁴ are as hereinbefore defined and n, p and q are zero or two, via an intermediate of formula (IX): 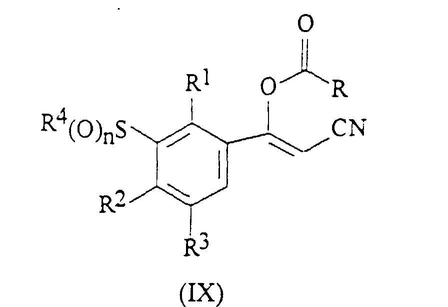 wherein R, R¹, R², R³ and R⁴ are as hereinbefore defined and n, p and q are zero or two. The formation and rearrangement of the intermediate of formula (IX) may be carried under the same conditions as described above for the formation and rearrangement of compounds of formula (VIII).

Intermediates in the preparation of compounds of formula (I) may be prepared by the application or adaptation of known methods.

Compounds of formula (II) or (III) in which R¹¹ represents hydrogen may be prepared by the reaction of a compound of formula (X): 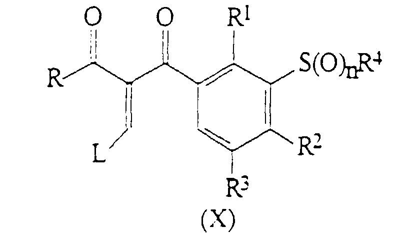 wherein R, R¹, R², R³, R⁴ and n are as hereinbefore defined and L is -OR⁷² or -N(R⁷²)₂ in which R⁷² is an alkyl group, with a salt of hydroxylamine in the presence of a base or acid acceptor. The reaction is generally carried out using hydroxylamine hydrochloride in the presence of sodium acetate or an organic base such as triethylamine. The reaction is preferably performed in a solvent. Suitable solvents include alcohols such as ethanol or inert solvents such as acetonitrile. The reaction is carried out at a temperature between room temperature and the boiling point of the solvent.

Compounds of formula (X) in which L represents -OR⁷² may be prepared by the reaction of a diketone of formula (XI): 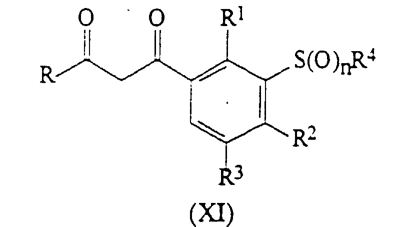 wherein R, R¹, R^{2,} R³, R⁴ and n are as hereinbefore defined, with an ortho ester, HC(OR⁷²)₃ wherein R⁷² is as hereinbefore defined. The reaction is generally carried out using triethyl orthoformate in the presence of an acid catalyst such as acetic anhydride. The reaction is carried out at a temperature between room temperature and the boiling point of the mixture.

Compounds of formula (X) in which L represents -N(R⁷²)₂ may be prepared by the reaction of a diketone of formula (XI) wherein R, R¹, R², R³, R⁴ and n are as hereinbefore defined with an amide acetal of formula (R⁷²)₂N-CH(OR⁷²)₂ wherein R⁷² is as hereinbefore defined. The reaction is optionally carried out in an inert solvent such as toluene at a temperature between room temperature and the boiling point of the mixture.

Compounds of formula (II) wherein R¹¹ represents an ester, nitrile or acyl group may be prepared by the reaction of a compound of formula (XII): 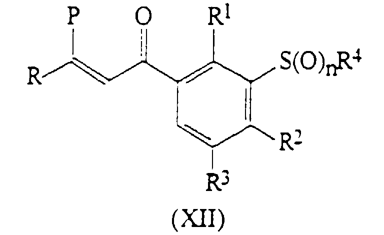 wherein R, R¹, R^{2,} R³, R⁴ and n are as hereinbefore defined and P is a leaving group such as N,N-dialkylamino, with a compound of formula R¹¹-C(Z)=NOH wherein R¹¹ represents an ester, nitrile or acyl group and Z is a halogen atom. Generally Z is a chlorine or bromine atom. The reaction is generally performed in an inert solvent such as toluene or dichloromethane either in the presence of a base such as triethylamine or a catalyst such as a 4 Angstrom molecular sieve or fluoride ion.

Compounds of formula (XII) may be prepared by the reaction of a compound of formula CH₂=C(R¹¹)(P), wherein R¹¹ and P are as hereinbefore defined, with a benzoyl chloride of formula (IV). The reaction is generally carried out in the presence of an organic base such as triethylamine in an inert solvent such as toluene or dichloromethane at a temperature between -20°C and room temperature.

Compounds of formula (II) or (III) wherein R¹¹ represents an ester, nitrile or acyl group may be prepared by the reaction of a compound of formula (XI) with a compound of formula R¹¹-C(Z)=NOH wherein R¹¹ represents an ester, nitrile or acyl group and Z is as hereinbefore defined. Generally Z is a chlorine or bromine atom. The reaction is generally performed in an inert solvent such as dichloromethane or acetonitrile in the presence of a base. Examples of suitable bases are alkaline earth metal alkoxides such as magnesium methoxide and the reaction is carried out a temperature between room temperature and the reflux temperature of the mixture.

Compounds of formula (II) or (III) wherein R¹¹ represents an amide group may be prepared by the reaction of the corresponding compound of formula (II) or (III) in which R¹¹ represents an ester group, with ammonia or an amine. The reaction is carried out in a solvent or solvent mixture such as aqueous ethanol at a temperature between room temperature and the reflux temperature of the mixture.

Compounds of formula (III) in which R¹¹ represents hydrogen may be prepared by the reaction of a compound of formula (XIII): 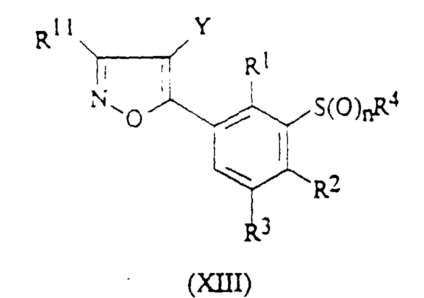 in which R¹¹ represents hydrogen and Y represents a carboxy group, or a reactive derivative thereof (such as a carboxylic acid chloride or carboxylic ester) or a cyano group, with an appropriate organometallic reagent such as a Grignard reagent or an organolithium reagent, to introduce the group -COR into the 4-position of the isoxazole ring. The reaction is generally carried out in an inert solvent such as ether or tetrahydrofuran, at a temperature from 0°C to the reflux temperature of the solvent.

Compounds of formula (III) in which R¹¹ is an ester, nitrile or acyl group may be prepared by the reaction of a compound of formula (XIV): 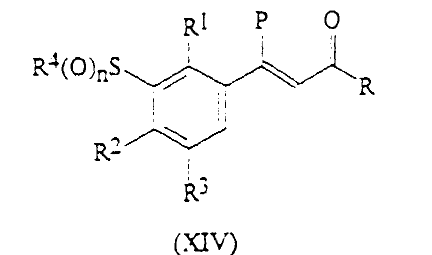 wherein R¹, R², R³, R⁴ and n are as hereinbefore defined and P is a leaving group such as N,N-dialkylamino with a compound of formula R¹¹C(Z) =N-OH wherein Z is as hereinbefore defined and R¹¹ is an ester, nitrile or acyl group. Generally Z is chlorine or bromine. The reaction is generally performed in an inert solvent such as toluene or dichloromethane either in the presence of a base such as triethylamine or a catalyst such as a 4Å molecular sieve or fluoride ion.

Compounds of formula (XIII) in which R¹¹ is a hydrogen atom and Y is -CO₂-alkyl or -CN may be prepared by the reaction of a compound of formula (XV): 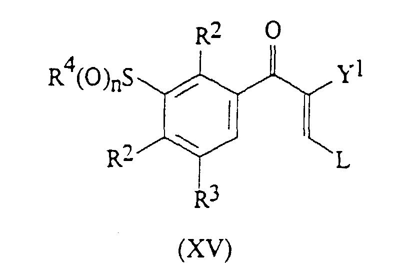 wherein R¹, R², R³, R⁴ and n are as hereinbefore defined and Y¹ represents CO₂-alkyl or -CN and L is as hereinbefore described, with a salt of hydroxylamine such as hydroxylame hydrochloride, in a solvent such as ethanol or acetonitrile, optionally in the presence of a base or acid acceptor such as triethylamine or sodium acetate.

Compounds of formula (XIII) in which R¹¹ represents hydrogen and Y represents a carboxylic acid or carboxylic acid chloride may be prepared from the corresponding compound of formula (XIII) in which R¹¹ represents hydrogen and Y represents a carboxylic ester group by the hydrolysis of said ester group and conversion, as necessary, of the acid thus obtained to the acid chloride, e.g. by heating with thionyl chloride.

Compounds of formula (XV) may be prepared by the reaction of a compound of formula (VII) or a ketoester of formula (XVI): 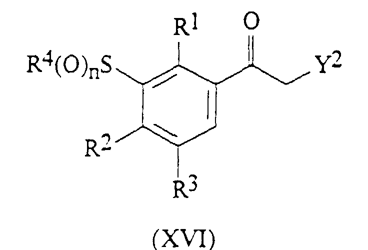 wherein R¹, R², R^{3,} R⁴ and n are as hereinbefore defined and Y² represents -CO₂-alkyl, with either a trialkyl orthoformate (e.g. triethyl orthoformate) in the presence of acetic anhydride at the reflux temperature of the mixture or with a dialkylformamide dialkylacetal (e.g. dimethylformamide dimethylacetal) optionally in an inert solvent such as toluene at a temperature from room temperature to the reflux temperature of the mixture.

Compounds of formula (XIV) may be prepared by the reaction of a compound of formula (XVII): 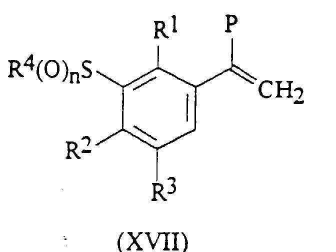 wherein R¹, R², R³, R⁴, n and P is as hereinbefore defined, with an acid chloride of formula (VI) wherein R is as hereinbefore defined, in an inert solvent such as dichloromethane or toluene, in the presence of a base such as triethylamine.

Acid chlorides of formula (IV) or (VI) are generally known or can be prepared from the corresponding carboxylic acid according to commonly accepted methods, for example by using thionyl chloride in chloroform at reflux.

Beta-ketonitriles of formula (V) may be prepared from acid chlorides of formula (VI) by a number of methods well known in the chemical literature. For example, see Krauss, et al, Synthesis, **1983**, 308, or Muth, et al, J. Org. Chem, **1960**, 25, 736. Alternatively beta-ketonitriles of formula (V) may be prepared by the reaction of an ester of formula R-CO₂Et, wherein R is as hereinbefore defined, with acetonitrile. This reaction is described in the literature, for example see the article by Abramovitch and Hauser, J. Am. Chem. Soc., **1942**, 64, 2720.

Beta-ketonitriles of formula (VII) may be prepared from benzoyl chlorides of formula (IV) or from ethyl benzoates of formula (XVIII): 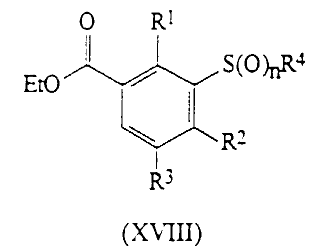 wherein R¹, R², R³, R⁴ and n are as hereinbefore defined, in a manner analogous to the preparation of beta-ketonitriles of formula (V) set forth above.

Compounds of formula (V), (XI), (XIV), (XVI), (XVII) and (XVIII) are known or may be prepared by the application and adaptation of known methods.

Interconversion of compounds of formula I or of intermediates is possible by the application or adaptation of known methods. Compounds in which n, p and q is one or two may be prepared by oxidation of the corresponding compounds in which n, p and q are zero or one using, for example, 3-chloroperoxybenzoic acid in an inert solvent such as dichloromethane at a temperature between -30°C and the boiling point of the solvent.

Compounds in which R¹, R² or R³ is a halogen atom may be prepared from corresponding compounds in which R¹, R² or R³ is replaced by an unsubstituted amino group by a Sandmeyer type reaction. This may be carried out using sodium nitrite in the presence of an acid such as hydrochloric acid or hydrobromic acid followed by treatment with for example copper (I) chloride or copper (I) bromide between room temperature and 80°C. Alternatively, diazotization may be carried out using an alkyl nitrite such as t-butyl nitrite in the presence of a halogenating agent such as copper (II) chloride or bromoform in an inert solvent such as acetonitrile.

Compounds in which R¹, R² or R³ is replaced by an unsubstituted amino group may be prepared by the reduction of compounds in which R¹, R² or R³ represents a nitro group, for example by means of tin (II) chloride and hydrochloric acid.

Compounds in which R¹, R² or R³ represents a cyano group may be prepared from compounds in which R¹, R² or R³ represents a group -CO₂R⁵ via hydrolysis to the corresponding carboxylic acid, in which R⁵ is replaced by hydrogen, conversion to a corresponding acid halide, for example by treatment with thionyl chloride, treatment with ammonia to give the amide, and dehydration, for example by means of phosphorus oxychloride. Compounds in which R¹, R² or R³ represents a nitro group may be prepared by the oxidation of compounds in which R¹, R² or R³ is replaced by an unsubstituted amino group, for example by means of reaction with trifluoroperacetic acid.

Agriculturally acceptable salts and metal complexes of compounds of formula (I) may be prepared by known methods.

The following examples illustrate the preparation of compounds of formula (I). In the present specification b.p. means boiling point, m.p. means melting point; cPr means cyclopropyl. Where the letters NMR appear, the characteristics of the proton nuclear magnetic resonance spectrum follow. Unless otherwise specified the percentages are by weight.

### EXAMPLE 1

Triethylamine (0.5g) was added to a solution of 5-cyclopropyl-4-(4-methyl-3-methylsulphenylbenzoyl)isoxazole (0.66g) in dichloromethane. The mixture was stirred at room temperature overnight. Further triethylamine (0.2g) was added and the mixture was stirred for a further 24 hours. Hydrochloric acid (2M) was added and the layers were separated. The organic layer was washed with aqueous sodium chloride solution, dried (Na₂SO₄) and filtered. The filtrate was evaporated to dryness to give 2-cyano-3-cyclopropyl-1-(4-methyl-3-methylsulphenylphenyl)propan-1,3-dione (compound 1, 0.12g) as an orange gum which solidified, m.p. 64.7-67.9°C.

### EXAMPLE 2

To a solution of sodium (0.05g) in ethanol (10ml) was added 5-cyclopropyl-4-(4-chloro-3-ethylsulphenyl-2-methylsulphenylbenzoyl)isoxazole (0.43g). The mixture was stirred at room temperature for 2 hours. Hydrochloric acid (2M) was added and the mixture extracted with ether and the layers separated. The organic layer was washed with water, dried (anhydrous sodium sulphate) and filtered. The filtrate was evaporated to dryness to give a creamy solid. The solid was triturated with ether to give 2-cyano-3-cyclopropyl-1-(4-chloro-3-ethylsulphenyl-2-methylsulphenylphenyl)propan-1,3-dione (compound 4, 0.15g) as a white solid, m.p. 63-65°C.

By proceeding in a similar manner the following compounds of formula (I) were prepared from the appropriately substituted starting materials.

| **Cpd** | **R** | **R**^{**1**} | **R**^{**2**} | **R**^{**3**} | **R**^{**4**} | **n** | **mp/°C** |
|---|---|---|---|---|---|---|---|
| 7 | cPr | Me | Cl | H | Me | 0 | 133.4-133.9 |
| 8 | cPr | CF₃ | SMe | H | Me | 0 | 115.0-116.0 |
| 9 | cPr | OMe | Cl | H | Me | 2 | 101-102 |
| 10 | cPr | SMe | Cl | H | nPr | 0 | 65.1-66.3 |

### EXAMPLE 3

Triethylamine (0.5g) was added to a solution of 5-(4-methyl-3-methylsulphonylphenyl)-4-cyclopropylcarbonylisoxazole (0.74g) in dichloromethane. The mixture was stirred overnight. Hydrochloric acid (2M) was added and the layers separated. The organic layer was washed with aqueous sodium chloride solution, dried (anhydrous magnesium sulphate) and filtered. The filtrate was evaporated to dryness to give 2-cyano-3-cyclopropyl-1-(4-methyl-3-methylsulphonylphenyl)propan-1,3-dione (compound 2, 0.55g) as a solid, m.p. 133.6-135.6

By proceeding in a similar manner the following compounds of formula (I) were prepared from the appropriately substituted starting materials:

Compound 3: 2-cyano-3-cyclopropyl-1-(4-methyl-3-methylsulphinylphenyl)propan-1,3-dione, m.p. 104.3°C, starting from 5-(4-methyl-3-methylsulphinylphenyl)-4-cyclopropylcarbonyl isoxazole.

Compound 6: 2-cyano-3-cyclopropyl-1-(4-chloro-3-methylsulphonyl-phenyl)propan-1,3-dione, m.p. 115.8 - 116.7°C, starting from 5-(4-chloro-3-methylsulphonylphenyl)-4-cyclopropylcarbonyl isoxazole.

### EXAMPLE 4

Triethylamine (0.5ml) was added to a solution of a 1:1 mixture of 5-cyclopropyl-4-(4-methoxy-3-methylsulphonylbenzoyl)isoxazole and 5-(4-methoxy-3-methylsulphonylphenyl)-4-cyclopropyl carbonyl isoxazole (0.65g) in dichloromethane. The mixture was stirred at room temperature for 2 hours. Hydrochloric acid (2M) was added and the layers separated. The organic layer was washed with aqueous sodium chloride solution, dried (anhydrous sodium sulphate) and filtered. The filtrate was evaporated to dryness to give 2-cyano-3-cyclopropyl-1-(4-methoxy-3-methysulphonylphenyl)propan-1,3-dione (compound 5, 0.5g) as a beige solid, m.p. 151-153.1°C.

### REFERENCE EXAMPLE 1

A mixture of 3-cyclopropyl-2-(N,N-dimethylaminomethylene)-1-(4-methyl-3-methysulphenylphenyl)propan-1,3-dione (10.6g) and hydroxylamine hydrochloride (2.92g) in ethanol was stirred at room temperature overnight. Water was added and the mixture was evaporated to remove the ethanol. It was extracted with ethyl acetate, washed with aqueous sodium chloride solution, dried (sodium sulphate) and filtered. The filtrate was evaporated to dryness and the residue was purified by chromatography on silica eluted with a mixture of ether and cyclohexane to give 5-cyclopropyl-4-(4-methyl-3-methylsulphenylbenzoyl)isoxazole (2.77g) as a white solid, m.p. 85.5-86.1°C.

By proceeding in a similar manner the following compounds were prepared from the appropriately substituted starting materials:-
5-(4-methyl-3-methylsulphenylphenyl)-4-cyclopropylcarbonyl isoxazole, m.p. 78.9-79.9°C, from 3-cyclopropyl-2-(N,N-dimethylaminomethylene)-1-(4-methyl-3-methylsulphenylphenyl)propan-1,3-dione;
A mixture (1:1) of 4-(4-methoxy-3-methylsulphonylbenzoyl)-5-cyclopropylisoxazole and 5-(4-methoxy-3-methylsulphonylphenyl)-4-cyclopropylcarbonyl isoxazole, from 3-cyclopropyl-2-(N,N-dimethylaminomethylene)-1-(4-methoxy-3-methylsulphonylphenyl)propan-1,3-dione.

### REFERENCE EXAMPLE 2

Sodium acetate (0.98g) was added to a mixture of 1-[4-chloro-3-(ethylsulphenyl)-2-(methylsulphenyl)phenyl]-3-cyclopropyl-2-ethoxymethylenepropan-1,3,-dione (3.4g) and hydroxylamine hydrochloride (0.83g) in ethanol and the mixture was stirred at room temperature overnight. The solid which separated was filtered and washed thoroughly with water and dried to give 4-[4-chloro-3-(ethylsulphenyl)-2-(methylsulphenyl)benzoyl)-5-cyclopropylisoxazole (1.45g) as a yellow solid m.p. 105-106°C.

By proceeding in a similar manner the following compounds of formula II above in which R¹¹ is hydrogen were prepared from the appropriately substituted starting materials.

| **R** | **R**^{**1**} | **R**^{**2**} | **R**^{**3**} | **R**^{**4**} | **n** | **mp/°C** |
|---|---|---|---|---|---|---|
| cPr | Me | Cl | H | Me | 0 | 83 |
| cPr | CF₃ | SMe | H | Me | 0 | 96-97 |
| cPr | OMe | Cl | H | Me | 0 | 75.1 |
| cPr | SMe | Cl | H | Pr | 0 | 67-69 |

### REFERENCE EXAMPLE 3

3-chloroperoxybenzoic acid (3.55g) was added to a stirred cooled solution of 4-(4-chloro-2-methoxy-3-methylsulphenylbenzoyl)-5-cyclopropylisoxazole (2.66g) in dichloromethane at 2°C. The mixture was stirred for 1 hour. The mixture was then filtered and washed with water, aqueous sodium metabisulphite, water. dried (anhydrous magnesium sulphate) and filtered. The filtrate was evaporated to dryness and the residue purified by dry column chromatography on silica eluting with a mixture of hexane and ethyl acetate (7:3) to give a white solid. This solid was dissolved in ethyl acetate and washed with saturated sodium bicarbonate, water, dried (anhydrous magnesium sulphate) and filtered. The filtrate was evaporated to dryness to give 4-(4-chloro-2-methoxy-3-methylsulphonylbenzoyl)-5-cyclopropylisoxazole (1.3g) as a white solid, m.p. 133.2°C.

By proceeding in a similar manner the following compounds were prepared from the appropriately substituted starting materials:-
5-(4-methyl-3-methylsulphonylphenyl)-4-cyclopropylcarbonyl isoxazole, m.p.110.8-112.2°C starting from 5-(4-methyl-3-methylsulphenylphenyl)-4-cyclopropylcarbonylisoxazole;
5-(4-methyl-3-methylsulphinylphenyl)-4-cyclopropylcarbonyl isoxazole, m.p.103.8-105.8°C starting from 5-(4-methyl-3-methylsulphenylphenyl)-4-cyclopropylcarbonylisoxazole;
5-(4-chloro-3-methylsulphonylphenyl)-4-cyclopropylcarbonylisoxazole, m.p. 169.2-172.6 from 5-(4-chloro-3-methylsulphenylphenyl)4-cyclopropylcarbonylisoxazole.

### REFERENCE EXAMPLE 4

Sodium acetate (3.0g) was added to a mixture of 3-cyclopropyl-2-ethoxymethylene 1-(4-chloro-3-methylsulphenylphenyl)propan-1,3-dione (12.3g) and hydroxylamine hydrochloride (3.0g) in ethanol and the mixture was stirred overnight at room temperature. The mixture was filtered and the solid was washed with ethanol and then water and dried to give 5-(4-chloro-3-methylsulphenyl)-4-cyclopropylcarbonylisoxazole (3.3g) as a white solid, m.p. 114.8-115.3°C.

### REFERENCE EXAMPLE 5

Dimethyl formamide dimethyl acetal (6.0 ml) was added to a solution of 3-cyclopropyl-1-(4-methyl-3-methylsulphenylphenyl)-propan-1,3-dione (8.71 g) in toluene. The mixture was stirred at room temperature overnight. Further dimethyl formamide dimethyl acetal (2.0 ml) was added and the mixture was stirred and heated at 50°C for 24 hours. It was cooled and evaporated to dryness to give 3-cyclopropyl-2-(N,N-dimethylaminomethylene)-1-(4-methyl-3-methylsulphenylphenyl)propan-1,3-dione (10.6 g) as a brown oil.

By proceeding in a similar manner 3-cyclopropyl-2-(N-methylaminomethylene)-1-(4-methoxy-3-methylsulphonylphenyl)propan-1,3-dione was prepared.

### REFERENCE EXAMPLE 6

A mixture of 1-[4,chloro-2-(methylsulphenyl)-3-(propylsulphenyl)phenyl]-3-cyclopropylpropan-1,3-dione (3.4g), triethylorthoformate (2.96g) and acetic anhydride (3.06g) were stirred and heated at reflux for 3 hours. The mixture was cooled and evaporated to dryness. The residue was dissolved in toluene and re-evaporated to give 1-[4-chloro-2-methylsulphenyl-3-propylsulphenylphenyl]-2-ethoxymethylene-3-cyclopropylpropan-1,3-dione (4.4g) as a red oil which was not purified further.

By proceeding in a similar manner and from the appropriately substituted starting materials the following compounds of formula (X) above in which L represents ethoxy were prepared:

| **R** | **R**^{**1**} | **R**^{**2**} | **R**^{**3**} | **R**^{**4**} | **n** |
|---|---|---|---|---|---|
| cPr | H | Cl | H | Me | 0 |
| cPr | Me | Cl | H | Me | 0 |
| cPr | CF₃ | SMe | H | Me | 0 |
| cPr | OMe | Cl | H | Me | 0 |
| cPr | SMe | Cl | H | Et | 0 |

### REFERENCE EXAMPLE 7

A suspension of magnesium (2.4 g) in methanol was warmed gently to initiate the reaction and t-butyl 3-cyclopropyl-3-oxopropionate (18.4 g) was added. The mixture was stirred for 0.75 hours then the methanol was evaporated off. Toluene was added and the mixture was re-evaporated to remove the last traces of methanol. The residue was suspended in acetonitrile and a solution of 4-methyl-3-methyl-3-methylsulphenylbenzoyl chloride (20.0 g) in acetonitrile was added. The mixture was stirred at room temperature overnight. Hydrochloric acid (2M) was added and the mixture was stirred for 1 hour. It was extracted with ethyl acetate, washed with aqueous sodium chloride solution, dried (magnesium sulphate) and filtered. The filtrate was evaporated to dryness to give an orange oil. This was dissolved in toluene and 4-toluenesulphonic acid (1.5 g) was added. The mixture was heated at reflux for 4 hours. It was cooled to room temperature and evaporated to dryness.

The residue was dissolved in ethyl acetate and washed with water, aqueous sodium chloride solution, dried magnesium sulphate and filtered. The filtrate was evaporated to dryness. The residue was purified by chromatography eluted with a mixture of ethyl acetate and cyclohexane to give 3-cyclopropyl-1-(4-methyl-3-methylsulphenylphenyl)propan-1,3-dione (9.99 g) as a brown oil, NMR (CDCl₃) 0.9-1.05(m,2H), 1.15-1.25(m,2H), 1.75-1.85(m,1H), 2.4(s,3H), 2.5(s,3H), 6.25(s,1H), 7.2(d,1H), 7.5(d,1H), 7.65(s,1H), 16.2-16.4(bs,1H).

By proceeding in a similar manner 3-cyclopropyl-1-(4-methoxy-3-methylsulphonylphenyl)-propan-1,3-dione was prepared from the appropriately substituted starting materials, NMR (CDCl₃) 0.95(m,2H), 1.2(m,2H), 1.8(m,1H), 3.7(s,3H), 4.05(s,3H), 6.3(s,1H), 7.1(d,1H), 8.15(d,1H), 8.45(d,1H), 16.3(bs,1H).

Benzoyl chlorides were prepared from the appropriately substituted benzoic acids by heating at reflux in thionyl chloride. The excess thionyl chloride is removed by evaporation and the residual benzoyl chlorides were used without further purification

### REFERENCE EXAMPLE 8

A mixture of magnesium (0.33g) and methanol containing carbon tetrachloride (0.1ml) was heated at reflux for half an hour. t-Butyl 3-cyclopropyl-3-oxopropionate (2.4g) was added dropwise and the resultant suspension was heated at reflux for 1 hour. It was cooled and evaporated to dryness. Toluene was added and the mixture re-evaporated to dryness. The residue was dissolved in toluene and 4-chloro-2-(methylsulphenyl)-3-(propylsulphenyl)benzoylchloride (3.24g) was added. The mixture was stirred at room temperature overnight. Hydrochloric acid (2M) was added and the mixture stirred for half an hour. The layers were separated and the organic layer was washed with water and dried by azeotropic removal of water using a Dean and Stark apparatus. 4-Toluenesulphonic acid (0.1g) was added and the mixture heated at reflux for 2 hours. It was cooled, washed with water, dried (anhydrous sodium sulphate) and filtered. The filtrate was treated with charcoal and filtered through hyflo silica and evaporated to give 1-[4-chloro-2-methylsulphenyl-3-propylsulphenylphenyl]-3-cypropylpropan-1,3-dione as a brown oil. NMR (CDCl₃) 1.0(m,5H), 1.2(m,2H), 1.6(m,2H), 1.7(m,1H), 2.45(s,3H), 2.9(t,2H), 6.05(s,1H), 7.25(d,1H), 7.55(d,1H), 16(bs, 1H).

By proceeding in a similar manner and from the appropriately substituted starting materials the following compounds of formula (XI) above were prepared:

| **R** | **R**^{**1**} | **R**^{**2**} | **R**^{**3**} | **R**^{**4**} | **n** | **NMR/m.p.** |
|---|---|---|---|---|---|---|
| cPr | Me | Cl | H | Me | 0 | (a) |
| cPr | CF₃ | SMe | H | Me | 0 | 96-97^{o}C |
| cPr | SMe | Cl | H | Et | 0 | (b) |
| cPr | OMe | Cl | H | Me | 0 | (c) |
| (a) NMR(CDCl₃) 0.9(m,2H), 1.2(m,2H), 1.7(m,1H), 2.35(s,3H), 2.75(s,3H), 5.8(s,1H), 7.35(m,2H). (b) NMR(CDCl₃) 0.95(m,2H), 1.15(m,2H), 1.7(m,1H), 2.4(s,3H), 2.95(q,2H), 5.9(s,1H), 7.2(d,1H), 7.4(d,1H), 16(bs,1H). (c) NMR(CDCl₃) 1.0(m,2H), 1.2(m,2H), 1.75(m,1H), 2.5(s,3H), 3.95(s,3H), 6.5(s,1H), 7.25(d,1H), 7.65(d,1H), 16.3(bs,1H). | | | | | | |

### REFERENCE EXAMPLE 9

To a stirred solution of sodium hydride (80% dispersion in oil) (4.20g) in tetrahydrofuran was added a solution of cyclopropylmethylketone (10.86g) and methyl (3-methylsulphenyl-4-chloro)benzoate (14.0g) in tetrahydrofuran dropwise over 2.5 hours. The mixture was stirred for a further 1.5 hours at reflux and then allowed to cool overnight. Hydrochloric acid (2M) was added. The mixture was extracted with ethyl acetate. The organic extract was washed with water, dried (anhydrous magnesium sulphate) and filtered. The filtrate was evaporated to dryness. The residue was purified by column chromatography on silica eluting with a mixture of cyclohexane and ethyl acetate (10:1) to give 3-cyclopropyl-1-(4-chloro-3-methylsulphenylphenyl)propan-1,3-dione (11.3g) as a solid, m.p. 76.5-79.4^{o}C.

### REFERENCE EXAMPLE 10

A solution of sodium nitrite (14.5 g) in water was added to a stirred, cooled suspension of 3-amino-4-methylbenzoic acid (30.23g) in a mixture of acetic acid and concentrated hydrochloric acid at a temperature between 0°C and 5°C. The mixture was stirred at 0-5°C for 1 hour and the resulting mixture was added to a stirred mixture of dimethyl disulphide and copper powder (0.25 g) in acetic acid while maintaining the temperature at about 35°C. Further copper powder (3 g) was added during the reaction in order to maintain the gas evolution. It was stirred for a further 1 hour then poured into water and the solid filtered off. It was treated with a mixture of ethyl acetate and cyclohexane, heated and the insoluble material was filtered off to give 4-methyl-3-methylsulphenylbenzoic acid (19.5 g) as a white solid, m.p. 174.6-175.2^{o}C.

By proceeding in a similar manner the following compounds were prepared from the appropriately substituted starting materials;
4-chloro-3-methylsulphenylbenzoic acid as a white solid, m.p. 208.0-209.3°C from 3-amino 4-chlorobenzoic acid;
4-methoxy-3-methylsulphenylbenzoic acid, from 3-amino 4-methoxybenzoic acid.

### REFERENCE EXAMPLE 11

To a stirred solution of 4-chloro-3-fluoro-2-methylsulphenylbenzoic acid (4.4g) and ethyl mercaptan (3.73g) in N,N-dimethyl formamide was added lithium hydroxide (3.35g). The mixture was stirred and heated at 80°C overnight. After this time a further portion of ethyl mercaptan (0.6g) and lithium hydroxide (1.26g) were added and the mixture heated at 80°C overnight. The mixture was cooled and poured into water. The mixture was acidified to pH 1 with hydrochloric acid (2M) and extracted with ether. The organic extract was washed with water, dried (anhydrous sodium sulphate) and filtered. The filtrate was evaporated to dryness to give after trituration with hexane 4-chloro-3-ethylsulphenyl-2-methylsulphenyl benzoic acid (2.57g) as a white solid, NMR(CDCl₃) 1.25(t,3H), 2.55(s,3H), 3.0(q,2H), 7.5(d,1H), 7.85(d,1H).

By proceeding in a similar manner 4-chloro-2-methylsulphenyl-3-propylsulphenylbenzoic acid, m.p. 92-93^{o}C was prepared from 4-chloro-3-fluoro-2-methylsulphenylbenzoic acid.

### REFERENCE EXAMPLE 12

To a stirred solution of 3-fluoro-4-methylsulphenyl-2-trifluoromethylbenzoic acid (2.5g) in N,N-dimethyl formamide was added sodium thiomethoxide (3.5g). The mixture was heated at 70°C for 5 hours and then 110^{o}C for 4 hours. After cooling the mixture was diluted with water and extracted with ether. The organic extract was washed with water, dried (anhydrous magnesium sulphate) and filtered. The filtrate was evaporated to dryness and the residue was purified by column chromatography on silica eluting with ethyl acetate to give 3,4-bismethylsulphenyl-2-trifluoromethylbenzoic acid (0.8g) as a beige solid, NMR (d₆ acetone) 1.3(s,3H), 1.4(s,3H), 7.5(d,1H), 8.1(d,1H).

### REFERENCE EXAMPLE 13

n-Butylithium (2.5M in hexane, 51ml) was added to a stirred, cooled solution of 6-bromo-2-fluoro-3-(methylsulphenyl)-benzotrifluoride (30.6g) in ether while maintaining the temperature below -70°C. The mixture was stirred at -78°C for 4 hours then poured onto carbon dioxide pellets. It was stirred for 2 hours and diluted with water. It was washed with ether and the aqueous layer was acidified and extracted with ether, washed with water, dried (MgSO₄) and filtered. The filtrate was evaporated to dryness to give 3-fluoro-4-(methylsulphenyl)-2-trifluoromethylbenzoic acid (23.4g) as a beige solid, NMR (DMSO-d₆) 3.14(s,3H), 7.99(d,1H), 8.19(t.1H).

### REFERENCE EXAMPLE 14

A solution of sodium nitrite (11.2g) in concentrated sulphuric acid was added to a stirred, cooled suspension of 4-bromo-2-fluoro-3-trifluoromethylaniline (40g) in glacial acetic acid while maintaining the temperature below 5°C. The mixture was stirred at 5°C for one and a half hours. The resultant mixture was added gradually to a mixture of dimethyl disulphide (20ml) and copper power (0.224g) in glacial acetic acid at 45°C. It was stirred and heated at 70°C for 3 hours. It was cooled, poured into water, extracted with ether, washed with water, dried (MgSO₄) and filtered. The filtrate was evaporated to dryness and purified by column chromatography eluted with petroleum spirit (b.p. 60-80°C) to give 6-bromo-2-fluoro-3-(methylsulphenyl)-benzotrifluoride (30.6g) as an orange oil, NMR (CDCl₃) 2.45(s,3H), 7.25(t,1H), 7.5(d,1H).

### REFERENCE EXAMPLE 15

A solution of N-bromosuccinimide (24.9g) in N,N-dimethyl formamide was added to a solution of 2-fluoro-3-trifluoromethylaniline (25g) in dimethyl formamide. The mixture was stirred for four and a half hours. It was poured into water and the oil was separated. The aqueous layers were extracted with ether and the combined organic layers were washed with water, dried (MgSO₄) and filtered. The filtrate was evaporated to dryness and the residue was distilled to give 4-bromo-2-fluoro-3-trifluoromethylaniline (27.44g) as an orange oil, b.p. 88-94°C/4mbar

### REFERENCE EXAMPLE 16

To a stirred solution of 1-chloro-3-fluorobenzene (10g) in tetrahydrofuran at -78°C under an inert atmosphere was added n-butylithium (37ml, 2.5M in hexane). The mixture was stirred at -78°C for 3 hours and then dimethyl disulphide (17.1 g) was added. The resultant mixture was allowed to warm to room temperature and stirred overnight. The mixture was quenched with water and extracted with ether. The organic extract was washed with water, dried (anhydrous magnesium sulphate) and filtered. The filtrate was evaporated to dryness to yield 2-chloro-6-fluorothioanisole (13.4g) as a clear oil, NMR (CDCl₃) 2.5(s,3H), 7.0(m.1H), 7.25(m,2H).

### REFERENCE EXAMPLE 17

To a stirred solution of 2-chloro-6-fluorothioanisole (13.4g) in tetrahydrofuran at -78°C under an inert atmosphere was added n-butyllithium (36ml, 2.5M in hexane). The mixture was stirred at -78°C for 3 hours and was then poured onto solid carbon dioxide (40g). The mixture was warmed to room temperature and evaporated. The residue was suspended in ether and washed with water. The aqueous extract was acidified to pH 1 with hydrochloric acid and extracted with ether. The organic extract was washed with water, dried (anhydrous magnesium sulphate) and filtered. The filtrate was evaporated to dryness and the residue triturated with hexane to give 4-chloro-2-fluoro-3-methylsulphenylbenzoic acid as a white solid, m.p. 183-185^{o}C.

### REFERENCE EXAMPLE 18

A suspension of 4-chloro-2-fluoro-3-methylsulphenylbenzoic acid (21.1g) in thionyl chloride (200ml) was heated at reflux for 5 hours. After cooling the mixture was evaporated to dryness. The residue was suspended in toluene and re-evaporated to dryness yielding 4-chloro-2-fluoro-3-methylsulphenylbenzoylchloride (22.9g) as a clear oil.

### REFERENCE EXAMPLE 19

To a solution of 2-amino-3-methylpropanol (17g) in dichloromethane at 0^{o}C was added a solution of 4-chloro-2-fluoro-3-methylsulphenylbenzoylchloride(22.9g) in dichloromethane. The mixture was stirred overnight at room temperature. The resultant suspension was filtered and the filtrate evaporated to dryness yielding N-(2,2-dimethyl-1-hydroxyethyl)-4-chloro-2-fluoro-3-methylsulphenylbenzamide (27.8g) as a brown gum, NMR (CDCl₃) 1.45(s,6H), 2.4(s,3H), 3.7(s,2H), 6.7(bd,1H), 7.25(dd,1H), 7.9(t,1H).

### REFERENCE EXAMPLE 20

To N-(2,2-dimethyl-1-hydroxyethyl)-4-chloro-2-fluoro-3-methylsulphenylbenzamide (27.8g) was added thionyl chloride (40g) with stirring. The mixture was then stirred for 1 hour and then added slowly to water and extracted with ether. The aqueous layer was basified with sodium hydroxide (2M) and extracted with dichloromethane. The organic extract was washed with water, dried (anhydrous magnesium sulphate) and filtered. The filtrate was evaporated to dryness and the residue purified by column chromatography on silica eluting with a mixture of ether and hexane (1:9) to give 2-(4-chloro-2-fluoro-3-methylsulphenylphenyl)-4,4-dimethyl-2-oxazoline (12.8g) as a yellow solid, m.p. 41.4-42.1^{o}C.

### REFERENCE EXAMPLE 21

To a suspension of magnesium (4.3g) and a crystal of iodine in ether at reflux was added methyl iodide (25.1g) dropwise. The resultant mixture was refluxed for 1 hour and then added to a solution of 2-(4-chloro-2-fluoro-3-methylsulphenylphenyl)-4,4-dimethyl-2-oxazoline (13.5g) in ether. The mixture was stirred overnight and then poured slowly onto a mixture of ice and hydrochloric acid (2M). The resultant mixture was neutralised with sodium hydroxide (2M) and extracted with ether. The organic extract was dried (anhydrous magnesium sulphate) and filtered. The filtrate was evaporated to dryness to give 2-(4-chloro-2-methyl-3-methylsulphenylphenyl)-4,4-dimethyl-2-oxazoline (12.4g) as a yellow oil, NMR (CDCl₃) 1.3(s,6H), 2.2(s,3H), 2.7(s,3H), 3.95(s,2H), 7.2(d,1H), 7.4(d,1H).

### REFERENCE EXAMPLE 22

2-(4-chloro-2-methyl-3-methylsulphenylphenyl)-4,4-dimethyl-2-oxazoline (12.4g) and a hydrochloric acid solution (364ml, 5M) were heated at reflux overnight. After cooling the mixture was extracted with ethyl acetate, dried (anhydrous magnesium sulphate) and filtered. The filtrate was evaporated to dryness to give 4-chloro-2-methyl-3-methylsulphenylbenzoic acid (10.0g) as a brown solid, m.p. 131.5°C.

### REFERENCE EXAMPLE 23

4-Chloro-2-fluoro-3-methylsulphenylbenzoyl chloride (19.9g) and methanol were stirred overnight at room temperature. The resultant mixture was evaporated to dryness and the residue dissolved in ether, washed with saturated sodium bicarbonate, water, dried (anhydrous magnesium sulphate) and filtered. The filtrate was evaporated to dryness to give methyl 4-chloro-2-fluoro-3-methylsulphenylbenzoate (19.2g) as a yellow oil, NMR (CDCl₃) 2.5(s,3H), 4.0(s,3H), 7.3(dd,1H), 7.8(t,1H).

### REFERENCE EXAMPLE 24

To a solution of methyl 4-chloro-2-fluoro-3-methylsulphenylbenzoate in tetrahydrofuran was added sodium methoxide (5.6g). The mixture was stirred overnight at room temperature. The mixture was diluted with water and extracted with ether. The organic extract was dried (anhydrous magnesium sulphate) and filtered. The filtrate was evaporated to dryness to give methyl 4-chloro-2-methoxy-3-methylsulphenylbenzoate (17. 1g) as a yellow oil, NMR(CDCl₃) 2.5(s,3H), 3.9(s,3H), 4.0(s,3H), 7.2(d,1H), 7.65(d,1H).

### REFERENCE EXAMPLE 25

A solution of methyl 4-chloro-2-methoxy-3-methylsulphenylbenzoate (6g) and sodium hydroxide (12g) in methanol and water were stirred at room temperature for 2 hours. The mixture was evaporated and the residue suspended in water and acidified to pH 1 with hydrochloric acid (2M). The solid was filtered and washed with water and dried in a vacuum oven to give 4-chloro-2-methoxy-3-methylsulphenylbenzoic acid (4.95g) as a white solid, m.p. 131-132°C.

### REFERENCE EXAMPLE 26

A solution of 4-methoxy-3-methylsulphenylbenzoic acid (27.3g) and sulphuric acid (10ml) in methanol was heated at reflux overnight. After cooling the mixture was evaporated, diluted with water and extracted with ethylacetate. The organic extract was washed with sodium bicarbonate solution, water and brine, dried (anhydrous magnesium sulphate) filtered and evaporated to give methyl 4-methoxy-3-methylsulphenylbenzoate (26.39g).

To a solution of methyl 4-methoxy-3-methylsulphenylbenzoate (13.7g) in dichloromethane was added 3-chloroperoxybenzoic acid (36g) (of 55% pure material). The mixture was stirred at room temperature overnight and then washed with saturated sodium bicarbonate, followed by brine, dried (anhydrous magnesium sulphate) and filtered. The filtrate was evaporated to dryness to give methyl 4-methoxy-3-methylsulphonylbenzoate (11.62g) as a white solid, m.p. 125.7-127.4°C.

According to a feature of the present invention, there is provided a method for controlling the growth of weeds (i.e. undesired vegetation) at a locus which comprises applying to the locus a herbicidally effective amount of at least one 2-cyano-1,3-dione derivative of formula (I) or an agriculturally acceptable salt, metal complex or enolic tautomeric form thereof. For this purpose, the 2-cyano-1,3-dione derivatives are normally used in the form of herbicidal compositions (i.e. in association with compatible diluents or carriers and/or surface active agents suitable for use in herbicidal compositions), for example as hereinafter described.

The compounds of formula (I) show herbicidal activity against dicotyledonous (i.e. broad-leafed) and monocotyledonous (e.g. grass) weeds by pre- and/or, post-emergence application.

By the term "pre-emergence application" is meant application to the soil in which the weed seeds or seedlings are present before emergence of the weeds above the surface of the soil. By the term "post-emergence application" is meant application to the aerial or exposed portions of the weeds which have emerged above the surface of the soil. For example, the compounds of formula (I) may be used to control the growth of
* broad-leafed weeds, for example, Abutilon theophrasti, Amaranthus retroflexus, Bidens pilosa, Chenopodium album, Galium aparine, Ipomoea spp. e.g. Ipomoea purpurea, Sesbania exaltata, Sinapis arvensis, Solanum nigrum and Xanthium strumarium, and
* grass weeds, for example Alopecurus myosuroides, Avena fatua, Digitaria sanguinalis, Echinochloa crus-galli, Eleusine indica and Setaria spp, e.g. Setaria faberii or Setaria viridis, and
* sedges, for example, Cyperus esculentus.

The amounts of compounds of formula (I) applied vary with the nature of the weeds, the compositions used, the time of application, the climatic and edaphic conditions and (when used to control the growth of weeds in crop-growing areas) the nature of the crops. When applied to a crop-growing area, the rate of application should be sufficient to control the growth of weeds without causing substantial permanent damage to the crop. In general, taking these factors into account, application rates between 0.01 kg and 5 kg of active material per hectare give good results. However, it is to be understood that higher or lower application rates may be used, depending upon the particular problem of weed control encountered.

The compounds of formula (I) may be used to control selectively the growth of weeds, for example to control the growth of those species hereinbefore mentioned, by pre- or post-emergence application in a directional or non-directional fashion, e.g. by directional or non-directional spraying, to a locus of weed infestation which is an area used, or to be used, for growing crops, for example cereals, e.g. wheat, barley, oats, maize and rice, soya beans, field and dwarf beans, peas, lucerne, cotton, peanuts, flax, onions, carrots, cabbage, oilseed rape, sunflower, sugar beet, and permanent or sown grassland before or after sowing of the crop or before or after emergence of the crop. For the selective control of weeds at a locus of weed infestation which is an area used, or to be used, for growing of crops, e.g. the crops hereinbefore mentioned, application rates between 0.01 kg and 4.0 kg, and preferably between 0.01 kg and 2 kg, of active material per hectare are particularly suitable.

The compounds of formula (I) may also be used to control the growth of weeds, especially those indicated above, by pre- or post-emergence application in established orchards and other tree-growing areas, for example forests, woods and parks, and plantations, e.g. sugar cane, oil palm and rubber plantations. For this purpose they may be applied in a directional or non-directional fashion (e.g. by directional or non-directional spraying) to the weeds or to the soil in which they are expected to appear, before or after planting of the trees or plantations at application rates between 0.25 kg and 5 kg, and preferably between 0.5 kg and 4 kg of active material per hectare.

The compounds of formula (I) may also be used to control the growth of weeds, especially those indicated above, at loci which are not crop-growing areas but in which the control of weeds is nevertheless desirable.

Examples of such non-crop-growing areas include airfields, industrial sites, railways, roadside verges, the verges of rivers, irrigation and other waterways, scrublands and fallow or uncultivated land, in particular where it is desired to control the growth of weeds in order to reduce fire risks. When used for such purposes in which a total herbicidal effect is frequently desired, the active compounds are normally applied at dosage rates higher than those used in crop-growing areas as hereinbefore described. The precise dosage will depend upon the nature of the vegetation treated and the effect sought.

Pre- or post-emergence application, and preferably pre-emergence application, in a directional or non-directional fashion (e.g. by directional or non-directional spraying) at application rates between 1 kg and 20 kg, and preferably between 5 and 10 kg, of active material per hectare are particularly suitable for this purpose.

When used to control the growth of weeds by pre-emergence application, the compounds of formula (I) may be incorporated into the soil in which the weeds are expected to emerge. It will be appreciated that when the compounds of formula (I) are used to control the growth of weeds by post-emergence application, i.e. by application to the aerial or exposed portions of emerged weeds, the compounds of formula (I) will also normally come into contact with the soil and may also then exercise a pre-emergence control on later-germinating weeds in the soil.

Where especially prolonged weed control is required, the application of the compounds of formula (I) may be repeated if required.

According to a further feature of the present invention, there are provided compositions suitable for herbicidal use comprising one or more of the 2-cyano-1,3-dione derivatives of formula (I) or an agriculturally acceptable salt, metal complex or enolic tautomeric form thereof in association with, and preferably homogeneously dispersed in, one or more compatible herbicidally-acceptable diluents or carriers and/or surface active agents [i.e. diluents or carriers and/or surface active agents of the type generally accepted in the art as being suitable for use in herbicidal compositions and which are compatible with compounds of formula (I)]. The term "homogeneously dispersed" is used to include compositions in which the compounds of formula (I) are dissolved in other components. The term "herbicidal compositions" is used in a broad sense to include not only compositions which are ready for use as herbicides but also concentrates which must be diluted before use. Preferably, the compositions contain from 0.05 to 90% by weight of one or more compounds of formula (I).

The herbicidal compositions may contain both a diluent or carrier and surface-active (e.g. wetting, dispersing, or emulsifying) agent. Surface-active agents which may be present in herbicidal compositions of the present invention may be of the ionic or non-ionic types, for example sulphoricinoleates, quaternary ammonium derivatives, products based on condensates of ethylene oxide with alkyl and polyaryl phenols, e.g. nonyl- or octyl-phenols, or carboxylic acid esters of anhydrosorbitols which have been rendered soluble by etherification of the free hydroxy groups by condensation with ethylene oxide, alkali and alkaline earth metal salts of sulphuric acid esters and sulphonic acids such as dinonyl-and dioctyl-sodium sulphonosuccinates and alkali and alkaline earth metal salts of high molecular weight sulphonic acid derivatives such as sodium and calcium lignosulphonates and sodium and calcium alkylbenzene sulphonates.

Suitably, the herbicidal compositions according to the present invention may comprise up to 10% by weight, e.g. from 0.05% to 10% by weight, of surface-active agent but, if desired, herbicidal compositions according to the present invention may comprise higher proportions of surface-active agent, for example up to 15% by weight in liquid emulsifiable suspension concentrates and up to 25% by weight in liquid water soluble concentrates.

Examples of suitable solid diluents or carriers are aluminium silicate, talc, calcined magnesia, kieselguhr, tricalcium phosphate, powdered cork, adsorbent carbon black and clays such as kaolin and bentonite. The solid compositions (which may take the form of dusts, granules or wettable powders) are preferably prepared by grinding the compounds of formula (I) with solid diluents or by impregnating the solid diluents or carriers with solutions of the compounds of formula (I) in volatile solvents, evaporating the solvents and, if necessary, grinding the products so as to obtain powders. Granular formulations may be prepared by absorbing the compounds of formula (I) (dissolved in suitable solvents, which may, if desired, be volatile) onto the solid diluents or carriers in granular form and, if desired, evaporating the solvents, or by granulating compositions in powder form obtained as described above. Solid herbicidal compositions, particularly wettable powders and granules, may contain wetting or dispersing agents (for example of the types described above), which may also, when solid, serve as diluents or carriers.

Liquid compositions according to the invention may take the form of aqueous, organic or aqueous-organic solutions, suspensions and emulsions which may incorporate a surface-active agent. Suitable liquid diluents for incorporation in the liquid compositions include water, glycols, tetrahydrofurfuryl alcohol, acetophenone, cyclohexanone, isophorone, toluene, xylene, mineral, animal and vegetable oils and light aromatic and naphthenic fractions of petroleum (and mixtures of these diluents). Surface-active agents, which may be present in the liquid compositions, may be ionic or non-ionic (for example of the types described above) and may, when liquid, also serve as diluents or carriers.

Powders, dispersible granules and liquid compositions in the form of concentrates may be diluted with water or other suitable diluents, for example mineral or vegetable oils, particularly in the case of liquid concentrates in which the diluent or carrier is an oil, to give compositions ready for use.

When desired, liquid compositions of the compound of formula (I) may be used in the form of self-emulsifying concentrates containing the active substances dissolved in the emulsifying agents or in solvents containing emulsifying agents compatible with the active substances, the simple addition of water to such concentrates producing compositions ready for use.

Liquid concentrates in which the diluent or carrier is an oil may be used without further dilution using the electrostatic spray technique.

Herbicidal compositions according to the present invention may also contain, if desired, conventional adjuvants such as adhesives, protective colloids, thickeners, penetrating agents, stabilisers, sequestering agents, anti-caking agents, colouring agents and corrosion inhibitors. These adjuvants may also serve as carriers or diluents.

Unless otherwise specified, the following percentages are by weight. Preferred herbicidal compositions according to the present invention are
* aqueous suspension concentrates which comprIe from 10 to 70% of one or more compounds of formula (I), from 2 to 10% of surface-active agent, from 0.1 to 5% of thickener and from 15 to 87.9% of water,
* wettable powders which comprise from 10 to 90% of one or more compounds of formula (I), from 2 to 10% of surface-active agent and from 8 to 88% of solid diluent or carrier,
* soluble powders which comprise from 10 to 90% of one or more compounds of formula (I), from 2 to 40% of sodium carbonate and from 0 to 88% of solid diluent
* liquid water soluble concentrates which comprise from 5 to 50%, e.g. 10 to 30%, of one or more compounds of formula (I), from 5 to 25% of surface-active agent and from 25 to 90%, e.g. 45 to 85%, of water miscible solvent, e.g. dimethylformamide, or a mixture of water-miscible solvent and water,
* liquid emulsifiable suspension concentrates which comprise from 10 to 70% of one or more compounds of formula (I), from 5 to 15% of surface-active agent, from 0.1 to 5% of thickener and from 10 to 84.9% of organic solvent
* granules which comprise from 1 to 90%, e.g. 2 to 10% of one or more compounds of formula (I), from 0.5 to 7%, e.g. 0.5 to 2%, of surface-active agent and from 3 to 98.5%, e.g. 88 to 97.5%, of granular carrier and,
* emulsifiable concentrates which comprise 0.05 to 90%, and preferably from 1 to 60% of one or more compounds of formula (I), from 0.01 to 10%, and preferably from 1 to 10%, of surface-active agent and from 9.99 to 99.94%, and preferably from 39 to 98.99%, of organic solvent.

Herbicidal compositions according to the present invention may also comprise the compounds of formula (I) in association with, and preferably homogeneously dispersed in, one or more other pesticidally active compounds and, if desired, one or more compatible pesticidally acceptable diluents or carriers, surface-active agents and conventional adjuvants as hereinbefore described. Examples of other pesticidally active compounds which may be included in, or used in conjunction with, the herbicidal compositions of the present invention include herbicides, for example to increase the range of weed species controlled for example alachlor [2-chloro-2,6'-diethyl-N-(methoxy-methyl)-acetanilide], atrazine [2-chloro-4-ethylamino-6-isopropylamino-1,3,5-triazine], bromoxynil [3,5-dibromo-4-hydroxybenzonitrile], chlortoluron [N'-(3-chloro-4-methylphenyl)-N,N-dimethylurea], cyanazine [2-chloro-4-(1-cyano-1-methylethylamino)-ethylamino-1,3,5-triazine], 2,4-D [2,4-dichlorophenoxy-acetic acid], dicamba [3,6-dichloro-2-methoxybenzoic acid], difenzoquat [1,2-dimethyl-3,5-diphenylpyrazolium salts], flampropmethyl [methyl N-2-(N- benzoyl-3-chloro-4-fluoroanilino)-propionate], fluometuron [N'-(3-trifluoro-methylphenyl)-N,N-dimethylurea], isoproturon [N'-(4-isopropylphenyl)-N,N-dimethylurea], nicosulfuron [2-(4,6-dimethoxypyrimidin-2-yl-carbamoylsulfamoyl)-N,N-dimethylnicotinamide] insecticides, e.g. synthetic pyrethroids, e.g. permethrin and cypermethrin, and fungicides, e.g. carbamates, e.g. methyl N-(1-butyl-carbamoyl- benzimidazol-2-yl)carbamate, and triazoles e.g. 1-(4-chlorophenoxy)-3,3-dimethyl-1-(1,2,4-triazol-1-yl)-butan-2-one.

Pesticidally active compounds and other biologically active materials which may be included in, or used in conjunction with, the herbicidal compositions of the present invention, for example those hereinbefore mentioned, and which are acids, may, if desired, be utilized in the form of conventional derivatives, for example alkali metal and amine salts and esters.

According to a further feature of the present invention there is provided an article of manufacture comprising at least one of the 2-cyano-1,3-dione derivatives of formula (I) or an agriculturally acceptable salt, metal complex or enolic tautomeric form thereof or, as is preferred, a herbicidal composition as hereinbefore described, and preferably a herbicidal concentrate which must be diluted before use, comprising at least one of the 2-cyano-1,3-dione derivatives of formula (I) within a container for the aforesaid derivative or derivatives of formula (I), or a said herbicidal composition, and instructions physically associated with the aforesaid container setting out the manner in which the aforesaid derivative or derivatives of formula (I) or herbicidal composition contained therein is to be used to control the growth of weeds. The containers will normally be of the types conventionally used for the storage of chemical substances which are solid at normal ambient temperatures and herbicidal compositions particularly in the form of concentrates, for example cans and drums of metal, which may be internally lacquered, and plastics materials, bottles or glass and plastics materials and, when the contents of the container is a solid, for example granular, herbicidal compositions, boxes, for example of cardboard, plastics materials and metal, or sacks. The containers will normally be of sufficient capacity to contain amounts of the 2-cyano-1,3-dione derivative or herbicidal compositions sufficient to treat at least one acre of ground to control the growth of weeds therein but will not exceed a size which is convenient for conventional methods of handling. The instructions will be physically associated with the container, for example by being printed directly thereon or on a label or tag affixed thereto. The directions will normally indicate that the contents of the container, after dilution if necessary, are to be applied to control the growth of weeds at rates of application between 0.01 kg and 20 kg of active material per hectare in the manner and for the purposes hereinbefore described.

The following Examples illustrate herbicidal compositions according to the present invention:

### EXAMPLE C1

A wettable powder is formed from:
* active ingredient (compound 1): 50% w/w
* nonylphenol/ethylene oxide condensate containing 9 moles of ethylene oxide per mol of phenol: 5% w/w
* silicon dioxide of micro-fine particle size: 5% w/w
* synthetic magnesium silicate carrier: 40% w/w
by absorbing the condensate on the silicon dioxide, mixing with the other ingredients and grinding the mixture in a hammermill to give a wettable powder.

Similar wettable powders may be prepared as described above by replacing the 2-cyano-1,3-dione (compound 1) by other compounds of formula (I).

### EXAMPLE C2

An aqueous suspension concentrate is formed from:
* active ingredient (compound 1): 50% w/v
* nonylphenol/ethylene oxide condensate containing 9 moles of ethylene oxide per mol of phenol: 1 % w/v
* sodium salt of polycarboxylic acid: 0.2% w/v
* Ethylene glycol : 5% w/v
* polysaccaride xanthan gum thickener: 0.15% w/v
* water to 100% by volume
by intimately mixing the ingredients and grinding in a ball-mill for 24 hours.

Similar aqueous concentrates may be prepared as described above by replacing the 2-cyano-1,3-dione (compound 1) by other compounds of formula (I).

A representative compound of formula (I) has been used in herbicidal applications according to the following procedures.

### Method of use of herbicidal compounds:

### Herbicidal activity:

Appropriate quantities of the compound used to treat the plant was dissolved in acetone to give solutions equivalent to an application rate of up to 1000g of the compounds used to treat the plants per hectare (g/ha). These solutions were applied at 260 litres of spray fluid per hectare.
a) Pre-emergence application weed control.
Seeds (weeds or crops) were sown in loam soil pots.
The compound of the invention was applied to the soil surface as described above.
b) Post-emergence application weed control.
Weed species were grown until ready for spraying with the compound of the invention. The growth stage of the plants at spraying were as follows :

| 1) Broad-leafed weeds : | |
|---|---|
| Abutilon theophrasti | 1-2 leaves. |
| Amaranthus retroflexus | 1-2 leaves. |
| Galium aparine | 1-2 whorls. |
| Sinapis arvensis | 2 leaves. |
| Ipomoea purpurea | 1-2 leaves. |
| Xanthium strumarium | 2 leaves. |

| 2) Grass weeds : | |
|---|---|
| Alopecurus myosuroides | 2 leaves. |
| Avena fatua | 1-2 leaves. |
| Echinochloa crus-galli | 2-3 leaves. |
| Setaria viridis | 2-3 leaves. |

| 3) Sedges : | |
|---|---|
| Cyperus esculentus | 3 leaves. |

c) Crop tolerance
The compound of the invention was applied pre-emergence as in (a) or post emergence (3-leaf stage) to the following crops:-wheat, maize, rice, soya and cotton.

A single pot of each plant species was allocated to each treatment with unsprayed controls and controls sprayed with acetone alone.

After treatment, the pots were kept in the greenhouse and were watered overhead.

Visual assessment of phytotoxicity was made 17-20 days after spraying. Weed control results were expressed as the percentage reduction in growth or kill of the weeds, in comparison with the plants in the control pots. Crop tolerance was expressed as the percentage damage to crop.

When used at an application rate of 1 kg/ha or less, compounds 1 to 10 gave at least 90% control against one or more of the weed species listed above: these compounds also shows selectivity on one or more of the listed crops.

## Claims

1. A 2-cyano-1,3-dione derivative of formula I: 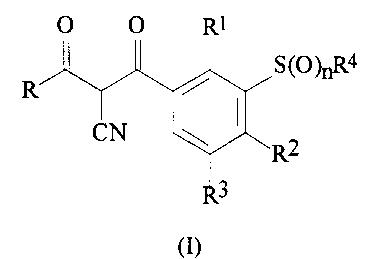 wherein:
R represents cyclopropyl;
R¹ represents:-
a hydrogen, chlorine or bromine atom, or
a straight- or branched-chain alkyl group containing up to 6 carbon atoms which is substituted by -OR⁵; or
a group selected from R⁵, nitro, cyano, -S(O)ₚR⁵, -OR⁵, -O(CH₂)ₘOR⁵ and -CO₂R⁵;
R² and R³, which may be the same or different, each represents: -
a halogen or hydrogen atom, or
a straight- or branched-chain alkyl group containing up to 6 carbon atoms which is substituted by -OR⁵; or
a group selected from R⁵, nitro, cyano, -OR⁵, -O(CH₂)ₘOR⁵, -S(O)_{q}R⁵ and -CO₂R⁵;
R⁴ and R⁵, which may be the same or different, each represents:-
a straight- or branched-chain alkyl group containing up to 6 carbon atoms which is optionally substituted by one or more halogen atoms which may be the same or different;
m is an integer from 1 to 3;
n is zero, 1 or 2; p is zero, 1 or 2; q is zero, 1 or 2;
with the proviso that when R¹ represents -S(O)ₚR⁵ at least one of the groups p and q is zero;
or an agriculturally acceptable salt, metal complex or enolic tautomeric form thereof.

2. A compound according to claim 1 wherein:
(a) R¹ represents:-
a hydrogen, chlorine or bromine atom, or
a group selected from -OR⁵, R⁵, nitro, or -SR⁵; and/or
(b) R² and R³, which may be the same or different, each represents:-
a halogen atom or hydrogen atom,
a straight- or branched-chain alkyl group containing up to 6 carbon atoms which is substituted by -OR⁵; or
a group selected from R⁵, -OR⁵, -O(CH₂)ₘOR⁵ where m is 2 or 3, -CO₂R⁵, or -S(O)_{q}R⁵,
provided that at least one of the groups R² and R³ represent hydrogen; and/or
(c) R⁴ represents:-
a straight- or branched-chain alkyl group containing up to 4 carbon atoms which is optionally substituted by one or more halogen atoms, which may be same or different; and/or
(d) R⁵ represents:-
a straight- or branched-chain alkyl group containing up to 4 carbon atoms which is optionally substituted by one or more halogen atoms, which may be the same or different; and
(e) 'halogen' represents chlorine, bromine or fluorine.

3. A compound according to claim 1 or 2 in which R³ represents a hydrogen atom.

4. A compound according to claim 1 in which:
R¹ represents chlorine, bromine, trifluoromethyl, -SR⁵, methoxy or methyl;
R² represents fluorine, chlorine, bromine, trifluoromethyl, -S(O)_{q}R⁵ or methyl,
R³ represents hydrogen;
R⁴ represents methyl, ethyl or isopropyl;
R⁵ represents methyl, ethyl or n-propyl; and
n is zero, one or two.

5. A compound according to claim 1 in which
R¹ represents hydrogen, methyl, trifluoromethyl, -SMe or methoxy;
R² represents methyl, methoxy, chlorine or -SMe,
R³ represents hydrogen;
R⁴ represents methyl, ethyl or propyl; and
n is zero, one or two.

6. A compound according to claim 1 which is:
2-cyano-3-cyclopropyl-1-(4-methyl-3-methylsulphenylphenyl)-propan-1,3-dione;
2-cyano-3-cyclopropyl-1-(4-methyl-3-methylsulphonylphenyl)propan-1,3-dione;
2-cyano-3-cyclopropyl-1-(4-methyl-3-methylsulphinylphenyl)propan-1,3-dione;
2-cyano-3-cyclopropyl-1-(4-chloro-3-ethylsulphenyl-2-methylsulphenylphenyl)propan-1,3-dione;
2-cyano-3-cyclopropyl-1-(4-methoxy-3-methylsulphonylphenyl)propan-1,3-dione;
2-cyano-3-cyclopropyl-1-(4-chloro-3-methylsulphonylphenyl)propan-1,3-dione;
2-cyano-3-cyclopropyl-1-(4-chloro-2-methyl-3-methylsulphenylphenyl)propan-1,3-dione;
2-cyano-3-cyclopropyl-1-(3,4-bismethylsulphenyl-2-trifluoromethylphenyl)propan-1,3-dione;
2-cyano-3-cyclopropyl-1-(4-chloro-2-methoxy-3-methylsulphonylphenyl)propan-1,3-dione; or
2-cyano-3-cyclopropyl-1 -(4-chloro-2-methylsulphenyl-3-propylsulphenylphenyl)propan-1,3-dione;
or an agriculturally acceptable salt, metal complex or enolic tautomeric form thereof.

7. A process for the preparation of a 2-cyano-1,3-dione derivative of formula (I) as defined in claim 1 which comprises:
(a) reacting a compound of formula (II) : 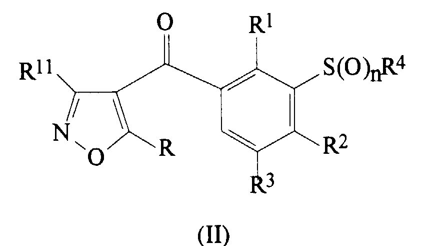 wherein R, R¹, R², R³, R⁴ and n are as defined in claim 1 and R¹¹ represents the hydrogen atom or a group selected from a carboxylic ester, amide, nitrile and acyl, with a base;
(b) reacting a compound of formula (III): 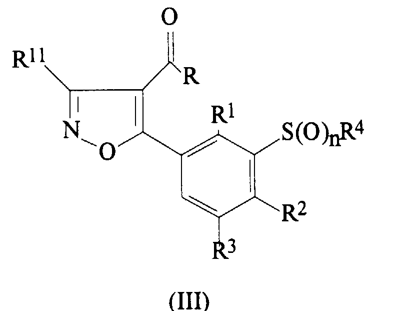 wherein R, R¹, R², R³, R⁴ and n are as defined in claim 1 and and R¹¹ is as defined above, with a base;
(c) where n, p and q are zero or two, reacting a benzoyl chloride of formula (IV): 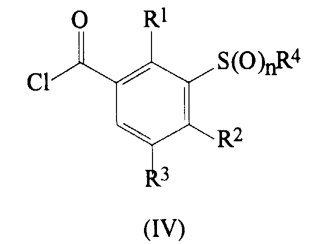 wherein R¹ R², R³ and R⁴ are as defined in claim 1 and n, p and q are zero or two, with a beta-ketonitrile of formula(V): 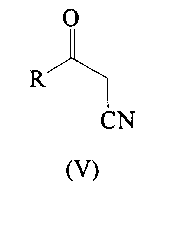 wherein R is as defined in claim 1;
(d) where n, p and q are zero or two, reacting an acid chloride of formula (VI): 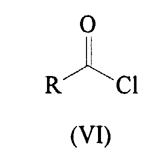 wherein R is as defined in claim 1, with a beta-ketonitrile of formula (VII): 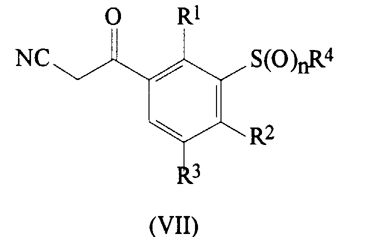 wherein R¹, R², R³ and R⁴ are as defined in claim 1 and n, p and q are zero or two;
(e) where n, p and q are zero or two, reacting a benzoyl chloride of formula (IV) above wherein R¹, R², R³ and R⁴ are as defined in claim 1 and n, p and q are zero or two, with a beta-ketonitrile of formula (V) above wherein R is as defined in claim 1, via an intermediate of formula (VIII): 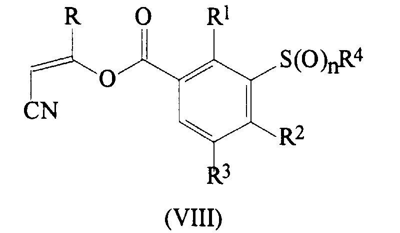 wherein R, R¹, R², R³, R⁴ are as defined in claim 1 and n, p and q are zero or two;
(f) where n, p and q are zero or two, reacting an acid chloride of formula (VI) as defined above wherein R is as defined in claim 1, with a beta-ketonitrile of formula (VII) as defined above wherein R¹, R², R³ and R⁴ are as defined in claim 1 and n, p and q are zero or two, via an intermediate of formula (IX): 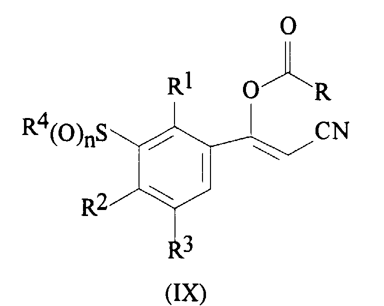 wherein R, R¹, R², R³, and R⁴ are as defined in claim 1 and n, p and q are zero or two; or
(g) where n, p and q are one or two ,oxidising the correponding compound of formula (I) in which n, p and q are zero or one;
optionally followed by the conversion of the compound thus obtained into an agriculturally aceptable salt or metal complex thereof.

8. A herbicidal composition which comprises as active ingredient a herbicidally effective amount of a 2-cyano-1,3-dione derivative of formula (I) as defined in any one of claims 1 to 6 or an agriculturally acceptable salt, metal complex or enolic tautomeric form thereof, in association with an agriculturally acceptable diluent or carrier and/or surface active agent.

9. A herbicidal composition according to claim 8 in the form of an aqueous suspension concentrate, a wettable powder, a water soluble or water dispersible powder, a liquid water soluble concentrate, a liquid emulsifiable suspension concentrate, a granule or an emulsifiable concentrate.

10. A method for controlling the growth of weeds at a locus which comprises applying to the locus a herbicidally effective amount of a 2-cyano-1,3-dione derivative of formula (I) as defined in any one of claims 1 to 6 or an agriculturally acceptable salt, metal complex or enolic tautomeric form thereof.

11. A method according to claim 10 in which the locus is an area used, or to be used, for the growing of crops and the compound is applied at an application rate from 0.01 kg to 4.0 kg per hectare.

## Patentansprüche

1. 2-Cyano-1,3-dion-Derivate der Formel I: 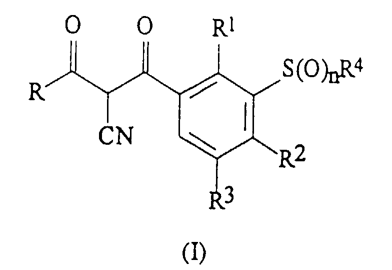 in der bedeuten:
R Cyclopropyl,
R¹ ein Wasserstoff-, Chlor- oder Bromatom oder
eine geradkettige oder verzweigte bis zu 6 Kohlenstoffatome enthaltende Alkylgruppe, die durch -OR⁵ substituiert ist, oder
eine unter R⁵, Nitro, Cyano, -S(O)ₚR⁵, -OR⁵, -O(CH₂)ₘOR⁵ und -CO₂R⁵ ausgewählte Gruppe,
R² und R³, die gleich oder voneinander verschieden sein können, jeweils:
ein Halogen- oder Wasserstoffatom oder
eine geradkettige oder verzweigte bis zu 6 Kohlenstoffatome enthaltende Alkyl-Gruppe, die durch -OR⁵ substituiert ist, oder
eine unter R⁵, Nitro, Cyano, -OR⁵, -O(CH₂)ₘOR⁵, -S(O)_{q}R⁵ und -CO₂R⁵ ausgewählte Gruppe,
R⁴ und R⁵, die gleich oder voneinander verschieden sein können, jeweils:
eine geradkettige oder verzweigte bis zu 6 Kohlenstoffatome enthaltende Alkylgruppe, die gegebenenfalls mit einem oder mehreren Halogenatomen, die gleich oder voneinander verschieden sein können, substituiert ist,
m eine ganze Zahl von 1 bis 3,
n 0, 1 oder 2, p 0, 1 oder 2, q 0, 1 oder 2,
mit der Maßgabe, daß mindestens eine der Gruppen p und q 0 ist, wenn R¹ -S(O)ₚR⁵ darstellt,
oder landwirtschaftlich akzeptable Salze, Metallkomplexe oder enoltautomere Formen davon.

2. Verbindung nach Anspruch 1, wobei bedeuten:
(a) R¹:
ein Wasserstoff-, Chlor- oder Bromatom oder
eine unter -OR⁵, R⁵, Nitro oder -SR⁵ ausgewählte Gruppe und/oder
(b) R² und R³, die gleich oder voneinander verschieden sein können, jeweils:
ein Halogenatom oder Wasserstoffatom,
eine geradkettige oder verzweigte bis zu 6 Kohlenstoffatome enthaltende Alkylgruppe, die durch -OR⁵ substituiert ist, oder
eine unter R⁵, -OR⁵, -O(CH₂)ₘOR⁵, wobei m 2 oder 3 ist, -CO₂R⁵ oder -S(O)_{q}R⁵ ausgewählte Gruppe,
mit der Maßgabe, daß mindestens eine der Gruppen R² und R³ Wasserstoff darstellt, und/oder
(c) R⁴:
eine geradkettige oder verzweigte bis zu 4 Kohlenstoffatome enthaltende Alkyl-Gruppe, die gegebenenfalls mit einem oder mehreren Halogenatomen, die gleich oder voneinander verschieden sein können, substituiert ist, und/oder
(d) R⁵:
eine geradkettige oder verzweigte bis zu 4 Kohlenstoffatome enthaltende Alkylgruppe, die gegebenenfalls mit einem oder mehreren Halogenatomen, die gleich oder voneinander verschieden sein können, substituiert ist, und
(e) "Halogen" Chlor, Brom oder Fluor.

3. Verbindung nach Anspruch 1 oder 2, wobei R³ ein Wasserstoffatom darstellt.

4. Verbindung nach Anspruch 1, wobei bedeuten:
R¹ Chlor, Brom, Trifluormethyl, -SR⁵, Methoxy oder Methyl,
R² Fluor, Chlor, Brom, Trifluormethyl, -S(O)_{q}R⁵ oder Methyl,
R³ Wasserstoff,
R⁴ Methyl, Ethyl oder Isopropyl,
R⁵ Methyl, Ethyl oder n-Propyl und
n 0, 1 oder 2.

5. Verbindung nach Anspruch 1, wobei bedeuten:
R¹ Wasserstoff, Methyl, Trifluormethyl, -SMe oder Methoxy,
R² Methyl, Methoxy, Chlor oder -SMe,
R³ Wasserstoff,
R⁴ Methyl, Ethyl oder Propyl und
n 0, 1 oder 2.

6. Verbindung nach Anspruch 1, nämlich:
2-Cyano-3-cyclopropyl-1-(4-methyl-3-methylsulfenylphenyl)propan-1,3-dion,
2-Cyano-3-cyclopropyl-1-(4-methyl-3-methylsulfonylphenyl)propan-1,3-dion,
2-Cyano-3-cyclopropyl-1-(4-methyl-3-methylsulfinylphenyl)propan-1,3-dion,
2-Cyano-3-cyclopropyl-1-(4-chlor-3-ethylsulfenyl-2-methylsulfenylphenyl)propan-1,3-dion,
2-Cyano-3-cyclopropyl-1-(4-methoxy-3-methylsulfonylphenyl)propan-1,3-dion,
2-Cyano-3-cyclopropyl-1-(4-chlor-3-methylsulfonylphenyl)propan-1,3-dion,
2-Cyano-3-cyclopropyl-1-(4-chlor-2-methyl-3-methylsulfenylphenyl)propan-1,3-dion,
2-Cyano-3-cyclopropyl-1-(3,4-bismethylsulfenyl-2-trifluormethylphenyl)propan-1,3-dion,
2-Cyano-3-cyclopropyl-1-(4-chlor-2-methoxy-3-methylsulfonylphenyl)propan-1,3-dion oder
2-Cyano-3-cyclopropyl-1-(4-chlor-2-methylsulfenyl-3-propylsulfenylphenyl)propan-1,3-dion
oder landwirtschaftlich akzeptable Salze, Metallkomplexe oder enoltautomere Formen davon.

7. Verfahren zur Herstellung von 2-Cyano-1,3-dion-Derivaten der in Anspruch 1 definierten Formel (I), bei dem:
(a) eine Verbindung der Formel (II): 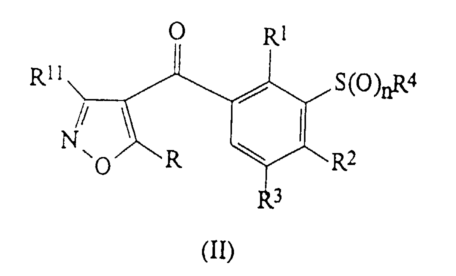 in der R, R¹, R², R³, R⁴ und n wie in Anspruch 1 definiert sind und R¹¹ ein Wasserstoffatom oder eine unter einem Carbonsäureester, Amid, Nitril und Acyl ausgewählte Gruppe darstellt, mit einer Base umgesetzt wird,
(b) eine Verbindung der Formel (III): 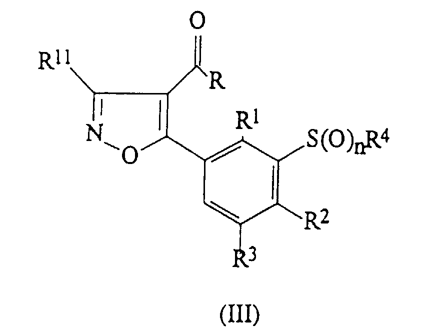 in der R, R¹, R², R³, R⁴ und n wie in Anspruch 1 definiert sind und R¹¹ das oben definierte bedeutet, mit einer Base umgesetzt wird,
(c) wenn n, p und q 1 oder 2 sind, ein Benzoylchlorid der Formel (IV): 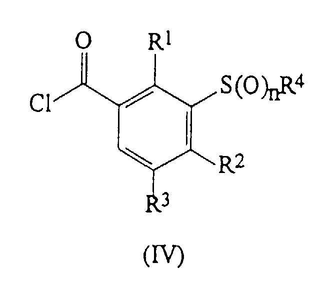 in der R¹, R², R³ und R⁴ wie in Anspruch 1 definiert und n, p und q 0 oder 2 sind, mit einem β-Ketonitril der Formel (V): 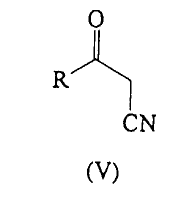 in der R wie in Anspruch 1 definiert ist, umgesetzt wird,
(d) wenn n, p und q 0 oder 2 sind, ein Säurechlorid der Formel (VI): 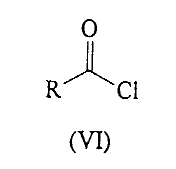 in der R wie in Anspruch 1 definiert ist, mit einem β-Ketonitril der Formel (VII): 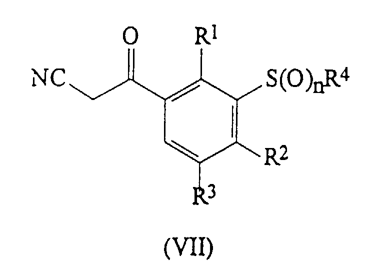 in der R¹, R², R³ und R⁴ wie in Anspruch 1 definiert und n, p und q 0 oder 2 sind, umgesetzt wird,
(e) wenn n, p und q 1 oder 2 sind, ein Benzoylchlorid der obigen Formel (IV), in der R¹, R², R³ und R⁴ wie in Anspruch 1 definiert und n, p und q 0 oder 2 sind, mit einem β-Ketonitril der obigen Formel (V), in der R wie in Anspruch 1 definiert ist, über ein Zwischenprodukt der Formel (VIII): 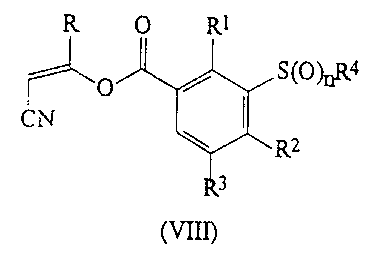 in der R, R¹, R², R³, R⁴ wie in Anspruch 1 definiert und n, p und q 0 oder 2 sind, umgesetzt wird,
(f) wenn n, p und q 0 oder 2 sind, ein Säurechlorid der oben definierten Formel (VI), in der R wie in Anspruch 1 definiert ist, mit einem β-Ketonitril der oben definierten Formel (VII), in der R¹, R², R³ und R⁴ wie in Anspruch 1 definiert und n, p und q 0 oder 2 sind, über ein Zwischenprodukt der Formel (IX): 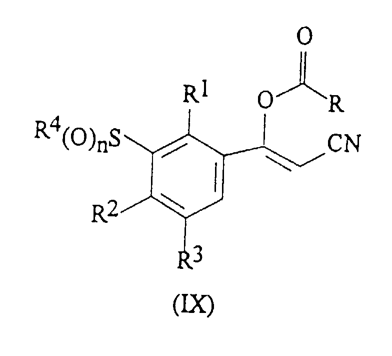 in der R, R¹, R², R³ und R⁴ wie in Anspruch 1 definiert und n, p und q 0 oder 2 sind, umgesetzt wird, oder
(g) wenn n, p und q 1 oder 2 sind, die entsprechende Verbindung der Formel (I), in der n, p und q 0 oder 1 sind, oxidiert wird,
worauf sich gegebenenfalls die Umwandlung der so erhaltenen Verbindung in landwirtschaftlich akzeptable Salze oder Metallkomplexe davon anschließt.

8. Herbicide Zusammensetzung, die als Wirkstoff eine herbicid wirksame Menge eines in einem der Ansprüche 1 bis 6 definierten 2-Cyano-1,3-dion-Derivats der Formel (I) oder eines landwirtschaftlich akzeptablen Salzes, Metallkomplexes oder einer enoltautomeren Form davon in Verbindung mit einem landwirtschaftlich akzeptablen Verdünnungsmittel oder Trägermittel und/oder grenzflächenaktiven Stoff enthält.

9. Herbicide Zusammensetzung nach Anspruch 8 in Form von wäßrigen Suspensionskonzentraten, benetzbaren Pulvern, wasserlöslichen oder in Wasser dispergierbaren Pulvern, flüssigen, in Wasser löslichen Konzentraten, flüssigen, emulgierbaren Suspensionskonzentraten, Granalien oder emulgierbaren Konzentraten.

10. Verfahren zur Bekämpfung des Unkräuterwachstums auf einer Örtlichkeit, bei dem auf die Örtlichkeit eine herbicid wirksame Menge eines in einem der Ansprüche 1 bis 6 definierten 2-Cyano-1,3-dion-Derivats der Formel (I) oder eines landwirtschaftlich akzeptablen Salzes, Metallkomplexes oder einer enoltautomeren Form davon ausgebracht wird.

11. Verfahren nach Anspruch 10, bei dem die Örtlichkeit eine Fläche ist, die zur Anzucht von Kulturpflanzen verwendet wird oder verwendet werden soll, und die Verbindung in einer Ausbringmenge von 0,01 kg bis 4,0 kg/ha ausgebracht wird.

## Revendications

1. Dérivé de 2-cyano-1,3-dione de formule (I) : 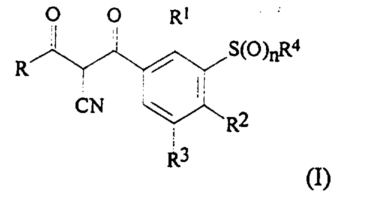 dans laquelle :
R représente un radical cyclopropyle ;
R¹ représente :
un atome d'hydrogène, un atome de chlore ou de brome, ou
un radical alkyle, linéaire ou ramifié, contenant de 1 à 6 atomes de carbone, substitué par un radical -OR⁵ ; ou
un radical choisi parmi les radicaux R⁵, nitro, cyano, -S(O)ₚR⁵, -OR⁵, -O(CH₂)ₘOR⁵ et -CO₂R⁵ ;
R² et R³, qui peuvent être identiques ou différents, représentent chacun :
un atome d'halogène ou un atome d'hydrogène ; ou
un radical alkyle, linéaire ou ramifié, contenant de 1 à 6 atomes de carbone, substitué par un radical -OR⁵ ; ou
un radical choisi parmi les radicaux R⁵, nitro, cyano, -OR⁵, -O(CH₂)ₘOR⁵, -S(O)_{q}R⁵, et -CO₂R⁵ ;
R⁴ et R⁵, qui peuvent être identiques ou différents, représentent chacun :
un radical alkyle, linéaire ou ramifié, contenant de 1 à 6 atomes de carbone, éventuellement substitué par un ou plusieurs atomes d'halogènes qui peuvent être identiques ou différents ;
m représente un nombre entier compris entre 1 et 3 ;
n représente 0, 1 ou 2 ;
p représente 0, 1 ou 2 ;
q représente 0, 1 ou 2 ;
avec la restriction que quand R¹ représente -S(O)ₚR⁵ au moins l'un des deux indices p et q représente zéro ;
ou un de ses sels, complexes métalliques ou formes énoliques tautomères acceptables en agriculture.

2. Composé selon la revendication 1 dans laquelle :
(a) R¹ représente :
un atome d'hydrogène, un atome de chlore ou de brome, ou
un radical choisi parmi les radicaux -OR⁵, R⁵, nitro, et -SR⁵ ; et/ou
(b) R² et R³, qui peuvent être identiques ou différents, représentent chacun :
un atome d'halogène ou un atome d'hydrogène,
un radical alkyle, linéaire ou ramifié, contenant de 1 à 6 atomes de carbone, substitué par un radical -OR⁵ ; ou
un radical choisi parmi les radicaux R⁵, -OR⁵, -O(CH₂)ₘOR⁵ dans lequel m représente 2 ou 3, -CO₂R⁵, et -S(O)_{q}R⁵,
avec la restriction que au moins l'un des radicaux R² et R³ représente un atome d'hydrogène ; et/ou
(c) R⁴ représente :
un radical alkyle, linéaire ou ramifié, contenant de 1 à 4 atomes de carbone, éventuellement substitué par un ou plusieurs atomes d'halogènes qui peuvent être identiques ou différents ; et/ou
(d) R⁵ représente :
un radical alkyle, linéaire ou ramifié, contenant de 1 à 4 atomes de carbone, éventuellement substitué par un ou plusieurs atomes d'halogènes qui peuvent être identiques ou différents ; et
(e) "halogène" représente le chlore, le brome ou le fluor.

3. Composé selon la revendication 1 ou 2 dans laquelle R³ représente un atome d'hydrogène.

4. Composé selon la revendication 1 dans laquelle :
R¹ représente le chlore, le brome, ou le radical trifluorométhyle, -SR⁵, méthoxy ou méthyle ;
R² représente le fluor, le chlore, le brome, ou le radical trifluorométhyle, -S(O)_{q}R⁵ ou méthyle ;
R³ représente un atome d'hydrogène ;
R⁴ représente le radical méthyle, éthyle ou isopropyle ;
R⁵ représente le radical méthyle, éthyle ou n-propyle ; et
n représente zéro, un ou deux.

5. Composé selon la revendication 1 dans laquelle :
R¹ représente un atome d'hydrogène, ou le radical méthyle, trifluorométhyle, -SMe ou méthoxy ;
R² représente le radical méthyle, méthoxy, -SMe ou un atome de chlore ;
R³ représente un atome d'hydrogène ;
R⁴ représente le radical méthyle, éthyle ou propyle ; et
n représente zéro, un ou deux.

6. Composé selon la revendication 1 qui est la :
2-cyano-3-cyclopropyl-1-(4-méthyl-3-méthylsulfénylphényl)propan-1,3-dione ;
2-cyano-3-cyclopropyl-1-(4-méthyl-3-méthylsulfonylphényl)propan-1,3-dione ;
2-cyano-3-cyclopropyl-1-(4-méthyl-3-méthylsulfinylphényl)propan-1,3-dione ;
2-cyano-3-cyclopropyl-1-(4-chloro-3-éthylsulfényl-2-méthylsulfénylphényl) propan-1,3-dione ;
2-cyano-3-cyclopropyl-1-(4-méthoxy-3-méthylsulfonylphényl)propan-1,3-dione ;
2-cyano-3-cyclopropyl-1-(4-chloro-3-méthylsulfonylphényl)propan-1,3-dione ;
2-cyano-3-cyclopropyl-1-(4-chloro-2-méthyl-3-méthylsulfénylphényl)propan-1,3-dione ;
2-cyano-3-cyclopropyl-1-(3,4-bisméthylsulfényl-2-trifluorométhylphényl) propan-1,3-dione ;
2-cyano-3-cyclopropyl-1-(4-chloro-2-méthoxy-3-méthylsulfonylphényl) propan- 1,3-dione ; et
2-cyano-3-cyclopropyl-1-(4-chloro-2-méthylsulfényl-3-propylsulfénylphényl) propan-1,3-dione ;
ou un de ses sels, complexes métalliques ou formes énoliques tautomères acceptables en agriculture.

7. Procédé de préparation des dérivés de 2-cyano-1,3-dione de formule (I) définie dans la revendication 1, qui comprend :
(a) la réaction d'un composé de formule (II) : 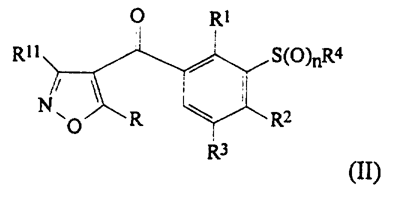 dans laquelle R, R¹, R², R³, R⁴ et n sont tels que définis dans la revendication 1 et R¹¹ représente un atome d'hydrogène ou un groupement choisi parmi les groupements ester carboxylique, amide, nitrile et acyle, avec une base ;
(b) la réaction d'un composé de formule (III) : 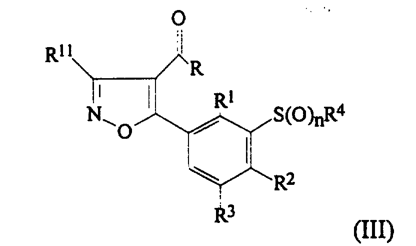 dans laquelle R, R¹, R², R³, R⁴ et n sont tels que définis dans la revendication 1 et R¹¹ est tel que défini précédemment, avec une base ;
(c) lorsque n, p et q représentent zéro ou deux, la réaction d'un chlorure de benzoyle de formule (IV) : 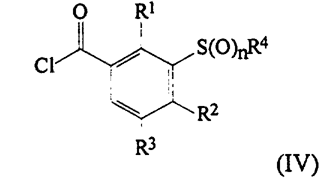 dans laquelle R¹, R², R³ et R⁴ sont tels que définis dans la revendication 1 et n, p et q représentent zéro ou deux, avec un béta-cétonitrile de formule (V) : 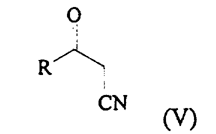 dans laquelle R est tel que défini dans la revendication 1 ;
(d) lorsque n, p et q représentent zéro ou deux, la réaction d'un chlorure d'acide de formule (VI) : 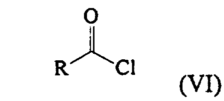 dans laquelle R est tel que défini dans la revendication 1, avec un béta-cétonitrile de formule (VII): 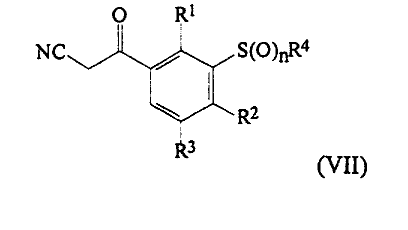 dans laquelle R¹, R², R³ et R⁴ sont tels que définis dans la revendication 1 et n, p et q représentent zéro ou deux ;
(e) lorsque n, p et q représentent zéro ou deux, la réaction d'un chlorure de benzoyle de formule (IV) définie précédemment dans laquelle R¹, R², R³ et R⁴ sont tels que définis dans la revendication 1 et n, p et q représentent zéro ou deux, avec un béta-cétonitrile de formule (V) définie précédemment dans laquelle R est tel aue défini dans la revendication 1, via un intermédiaire de formule (VIII): 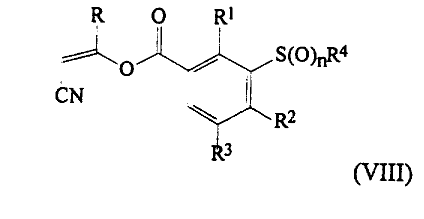 dans laquelle R, R¹, R², R³, R⁴ sont tels que définis dans la revendication 1 et n, p et q représentent zéro ou deux ;
(f) lorsque n, p et q représentent zéro ou deux, la réaction d'un chlorure d'acide de formule (VI) définie précédemment dans laquelle R est tel que défini dans la revendication 1, avec un béta-cétonitrile de formule (VII) définie précédemment dans laquelle R¹, R², R³ et R⁴ sont tels que définis dans la revendication 1 et n, p et q représentent zéro ou deux, via un intermédiaire de formule (IX) : 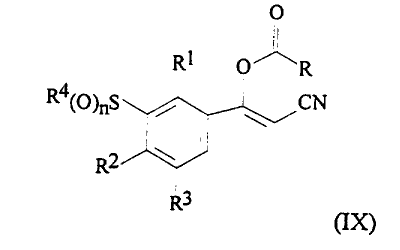 dans laquelle R, R¹, R², R³ et R⁴ sont tels que définis dans la revendication 1 et n, p et q représentent zéro ou deux ; ou
(g) lorsque n, p et q représentent un ou deux, l'oxydation du composé correspondant de formule (I) dans laquelle n, p et q représentent zéro ou un ;
éventuellement suivie de la conversion du composé ainsi obtenu en un de ses sels ou complexes métalliques acceptables en agriculture.

8. Composition herbicide comprenant comme matière active une quantité herbicide efficace d'un dérivé de 2-cyano-1,3-dione de formule (I) comme défini dans l'une quelconque des revendications 1 à 6 ou un sel, complexe métallique ou une forme énolique tautomère de celui-ci acceptable en agriculture, en association avec un diluant ou une support et/ou un agent tensioactif acceptable en agriculture.

9. Composition herbicide selon la revendication 8 qui se présente sous la forme d'un concentré en suspension aqueuse, d'une poudre mouillable, d'une poudre soluble dans l'eau ou dispersable dans l'eau, d'un concentré liquide soluble dans l'eau, d'un concentré en suspension liquide émulsionnable, de granulés ou d'un concentré émulsionnable.

10. Méthode de lutte contre la croissance des mauvaises herbes en un lieu comprenant l'application sur le dit lieu d'une quantité herbicide efficace d'un dérivé de 2-cyano-1,3-dione de formule (I) comme défini dans l'une quelconque des revendications 1 à 6, ou un sel, un complexe métallique ou une forme énolique tautomère de celui-ci acceptable en agriculture.

11. Méthode selon la revendication 10 dans laquelle le lieu est une surface utilisée, ou pouvant être utilisée, pour la croissance des cultures et le composé est utilisé à des doses d'application comprises entre 0,01 kg et 4,0 kg par hectare.
